(19) Europäisches Patentamt
European Patent Office
Office européen des brevets

(11) Veröffentlichungsnummer: **0 006 243**
**B1**

(12) # EUROPÄISCHE PATENTSCHRIFT

(45) Veröffentlichungstag der Patentschrift: **03.02.82**

(21) Anmeldenummer: **79102016.7**

(22) Anmeldetag: **18.06.79**

(51) Int. Cl.³: **G 03 C 7/36,**
C 07 C 103/82,
C 07 C 143/80,
C 07 C 147/12

(54) **Farbphotographisches Aufzeichnungsmaterial, farbphotographisches Verfahren zur Herstellung eines Gelbbildes, Gelbkuppler und Verfahren zur Herstellung der Gelbkuppler.**

(30) Priorität: **20.06.78 CH 6730/78**

(43) Veröffentlichungstag der Anmeldung:
**09.01.80 Patentblatt 80/1**

(45) Bekanntmachung des Hinweises auf die Patenterteilung:
**03.02.82 Patentblatt 82/5**

(84) Benannte Vertragsstaaten:
**BE CH DE FR GB IT**

(56) Entgegenhaltungen:
**FR - A - 848 246**
**FR - A - 2 375 626**
**US - E - 27 848**

(73) Patentinhaber: **CIBA-GEIGY AG**
**Patentabteilung Postfach**
**CH-4002 Basel (CH)**

(72) Erfinder: **Fryberg, Mario, Dr.**
**Dery-le-Mont**
**CH-1724 Praroman-le-Mouret (CH)**

(74) Vertreter: **Berg, Wilhelm, Dr.**
**Dr. Berg, Dipl.-Ing. Stapf, Dipl.-Ing. Schwabe, Dr.**
**Dr. Sandmair Patentanwälte Postfach 860245**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 006 243 B1

# Farbphotographisches Aufzeichnungsmaterial, farbphotographisches Verfahren zur Herstellung eines Gelbbildes, Gelbkuppler und Verfahren zur Herstellung der Gelbkuppler

Zur Erzeugung farbphotographischer Bilder werden bekanntlich belichtete Silberhalogenid-Emulsionschichten, die gleichzeitig Farbkuppler enthalten, mit einer aromatischen, primäre Aminogruppen enthaltenden Entwicklersubstanz entwickelt. Die oxydierte Entwicklersubstanz reagiert mit dem Farbkuppler unter Bildung eines Bildfarbstoffes, wobei dessen Menge von der Menge des entwickelten Silbers abhängig ist.

Im allgemeinen wird ein lichtempfindliches photographisches Mehrschichtenmaterial verwendet, das aus einer rotempfindlichen Schicht, die den Blaugrünkuppler enthält, einer grünempfindlichen Schicht, die den Purpurkuppler enthält, und einer blauempfindlichen Schicht, die ihrerseits den Gelbkuppler enthält, besteht. Bei der Farbentwicklung entstehen dann die entsprechenden Farbstoffe mit den Farben blaugrün, purpur und gelb.

Gewöhnlich werden als Blaugrünkuppler Phenole oder $\alpha$-Naphthole, als Purpurkuppler Pyrazolone und als Gelbkuppler Acylacetylamide eingesetzt. Die nach der Entwicklung gebildeten Farbstoffe sind dann Indophenole, Indamine oder Azomethine.

Als Strukturmerkmal besitzen die üblichen Gelbkuppler eine aktive Methylengruppe, wobei gegebenenfalls ein Wasserstoffatom durch eine während der Kupplungsreaktion abspaltbare Gruppe ersetzt sein kann. Im ersten Fall spricht man von Vieräquivalentkupplern, da vier Aequivalente Silberhalogenid zur Bildung des Bildfarbstoffes benötigt werden. Im zweiten Fall werden zur Erzeugung des entsphrechenden Bildfarbstoffes nur noch zwei Aequivalente Silberhalogenid verwendet (Zweiäquivalentkuppler). Diese bekannten Kuppler führen zu Bildfarbstoffen, die jeweils eine farbgebende Gruppierung (Azomethingruppierung) und eine Ballastgruppe enthalten. Obwohl die Ballastgruppen insofern von Bedeutung sind als sie beispielsweise einerseits für die Löslichkeit der Kuppler, andererseits auch für Diffusionsfestigkeit der Farbstoffe verantwortlich sind, so können sie jedoch auch die photographischen Eigenschaften des farbphotographischen Aufzeichnungsmaterials ungünstig beeinflussen (z.B. durch unerwünschte Lichtabsorption); ausserdem leisten sie keinen Beitrag zur Erhöhung der Farbausbeute des zu bildenden Bildfarbstoffs. Um diese Nachteile zu überwinden, kann man nun entweder die Ballastgruppen verkleinern (was jedoch wegen der an sie gestellten Forderungen kaum erfolgversprechend ist) oder man erhöht die Zahl der farbgebenden Gruppierungen pro Molelül. Durch diese Massnahme erhöht man die molare Farbbildungskapazität der Kuppler und erzeilt eine grössere Farbdichte, so dass die Einsatzmenge der Kuppler gesenkt werden kann, womit gleichzeitig auch die Menge der Ballastgruppen und damit ihr gegebenenfalls störender Einfluss auf das photographische Material vermindert werden kann. Farbkuppler dieser Art sind z.B. aus US—E—27848 2 mal 2- und 2 mal 4-Äquivalentkuppler), US—A—3 077 043 und DE—A—2 408 168 bekannt. Die Gebrauchseigenschaften dieser Kuppler sind jedoch noch nicht voll befriedigend.

Aufgabe der vorliegenden Erfindung ist es daher, neue farbphotographische Materialien mit verbesserten Eigenschaften bereitzustellen, in denen als Gelbkuppler Verbindungen eingesetzt werden, die pro Molekül drei oder vier farbgebende Gruppierungen und ein Brückenglied zwischen den farbgebenden Gruppierungen aufweisen.

Bei den erfindungsgemäss einzusetzenden Farbkupplern kann es sich sowohl um sog. 3 mal 2 oder 4 mal 2- als auch um 3 mal 4 oder 4 mal 4- Aequivalentkuppler handeln, d.h. um Verbindungen die pro Molekül 3 oder 4 reaktive, zur Farbbildung mit dem oxydierten Entwickler befähigte Stellen besiezen.

Gegenstand der vorliegenden Erfindung ist ein lichtempfindliches farbphotographisches Aufzeichnungsmaterial, das in mindestens einer Silberhalogenidemulsionsschicht mindestens einen Gelbkuppler mit mehreren während einer Kupplungsreaktion abspaltbaren Gruppen enthält, dadurch gekennzeichnet, dass der Kuppler der Formel

(1) 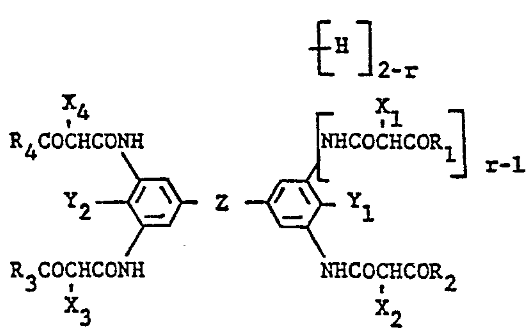

entspricht, worin

$R_1$, $R_2$, $R_3$ und $R_4$ Alkyl, Cycloalkyl oder Aryl,

$X_1$, $X_2$, $X_3$ und $X_4$ während einer Kupplungsreaktion abspaltbare Reste, und

$Y_1$ und $Y_2$ Halogen, Alkyl, Alkoxy, Alkylmercapto, —CN, —COOH, Carbalkoxy, —$NH_2$, —$NHR_5$,

—$NR_5R_6$ oder —$NHCOR_5$ sind, worin $R_5$ und $R_6$ Alkyl oder Phenyl sind,

Z ein geradkettiges Alkylen mit 1 bis 20 Kohlenstoffatomen, gegebenenfalls substituiert mit Alkyl, Hydroxyalkyl, Alkoxyalkyl, Aminoalkyl, Acylaminoalkyl oder Halogenalkyl mit je 1 bis 10 Kohlenstoffatomen im Alkylteil und 1 bis 5 Kohlenstoffatomen im Alkoxy- und Acylteil, Hydroxyl, Halogen, Alkoxy mit 1 bis 5 Kohlenstoffatomen, Amino (—$NH_2$), N-Alkylamino und N,N-Dialkylamino mit je 1 bis 5 Kohlenstoffatomen im Alkylteil, Mercapto (—SH) und/oder Alkylmercapto mit 1 bis 5 Kohlenstoffatomen, Z ferner Cycloalkylen mit 3 bis 12 Kohlenstoffatomen,

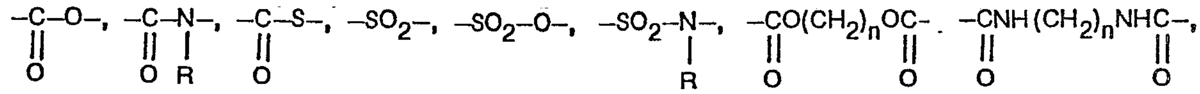

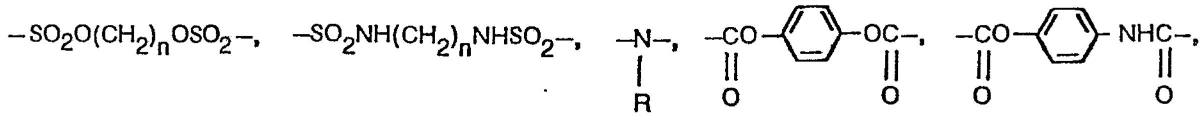

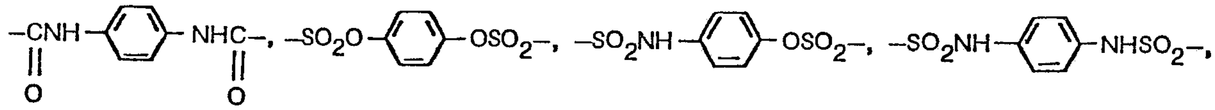

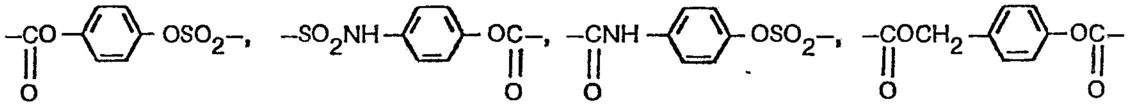 oder

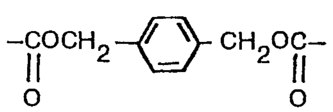 ist,

worin R Wasserstoff, Alkyl mit 1 bis 10 Kohlenstoffatomen, gegebenenfalls substituiert mit Halogen, Hydroxyl oder Acyl mit 2 bis 5 Kohlenstoffatomen und n eine ganze Zahl von 2 bis 20 ist, und r 1 oder 2 ist.

Gegenstand der Erfindung sind ausserdem ein farbphotographisches Verfahren zur Herstellung eines Gelbbildes durch Farbentwicklung eines belichteten Aufzeichnungsmaterials, das mindestens eine Verbindung der Formel (1) als Gelbkuppler enthält, die Verbindungen der Formel (1) sowie die Verwendung von Verbindungen der Formel (1) als Gelbkuppler in lichtempfindlichen farbphotographischen Aufzeichungsmaterialien.

Geeignete Alkylreste $R_1$, $R_2$, $R_3$ und $R_4$ in Verbindungen der Formel (1) können 1 bis 18 Kohlenstoffatome enthalten und geradkettig oder verzweigt sein, wie z.B. Methyl, Aethyl, Propyl, i-Propyl, Butyl, Isobutyl, tert.-Butyl, Amyl, tert.-Amyl (1,1-Dimethylpropyl), 1,1,3,3-Tetramethylbutyl, 1-Methyl-1-äthylpentyl, Hexyl, 1-Methylpentyl, Neopentyl, 1-, 2- oder 3-Methylhexyl, Heptyl, n-Octyl, tert.-Octyl, 2-Aethylhexyl, n-Nonyl, Isononyl, tert.-Nonyl, Decyl, tert.-Decyl, Undecyl; ferner Dodecyl, Tetradecyl, Hexadecyl und Octadecyl sowie die dazugehörigen Isomeren. Besonders geeignet sind geradkettige oder verzweigte Alkylreste mit 3 bis 10 Kohlenstoffatomen und von diesen sind tert. Alkylreste mit 4 bis 8 Kohlenstoffatomen bevorzugt.

Tert.-Butyl, 1,1,3,3-Tetramethylbutyl, 1-Methyl-1-äthylpentyl und 1,1-Dimethylpropyl sind besonders bevorzugte tertiäre Alkylreste.

Beispiele für Cycloalkyl sind solche mit 3 bis 12 Ringkohlenstoffatomen, wie z.B. Cyclopropyl, Cyclobutyl, Cyclopentyl und Cyclohexyl, ferner Cyclooctyl und Cyclododecyl, die gegebenenfalls substituiert sein können. Cycloalkyl schliesst auch Bi- und Tricycloalkyl, wie z.B. Norbornyl und Adamantyl ein. Cyclopentyl, Cyclohexyl und Adamantyl sind bevorzugt.

Arylreste sind insbesondere Phenyl oder substituiertes Phenyl, wobei als Substituenten (ein oder mehrere Substituenten) Halogen z.B. Fluor, Chlor oder Brom, Alkyl oder Alkoxy, vorzugsweise mit je 1 bis 4 Kohlenstoffatomen, wie z.B. Methyl, Aethyl, Propyl, i-Propyl, Butyl, tert. Butyl, Methoxy, Aethoxy, Propoxy, Butoxy infrage kommen, ferner Amino (—$NH_2$), Sulfo (—$SO_3H$), Alkylsulfonyl und Acylamino, wobei die beiden letzteren durch die Formeln —$SO_2R_{11}$ und —$NHCOR_{12}$— dargestellt werden können, worin $R_{11}$ Alkyl mit 1 bis 5 Kohlenstoffatomen, wie z.B. Methyl, Aethyl, Propyl, i-Propyl, Butyl oder Amyl und $R_{12}$ ebenfalls Alkyl mit 1 bis 5 Kohlenstoffatomen ist, wobei als spezielle Reste ebenfalls die für $R_{11}$ genannten gelten. Ein weiterer Substituent am Phenylring kann —$O(CH_2)_rO$— sein, worin r 1 oder 2 ist.

Phenyl substituiert mit Halogen und Alkyl oder Alkoxy mit je 1 bis 4 Kohlenstoffatomen ist als Arylrest bevorzugt. Die Reste $R_1$, $R_2$, $R_3$ und $R_4$ können gleich oder voneinander verschieden sein.

Als während der Kupplungsreaktion abspaltbare Reste $X_1$, $X_2$, $X_3$, und $X_4$, die gleich oder voneinander verschieden sein können, kommen Wasserstoff, Halogen, Alkoxy und Phenoxy, gegebenenfalls substituiert, stickstoffhaltige 5- oder 6-gliedrige Heterocyclen, die über ein Stickstoffatom an die Kupplungsstelle gebunden sind, die Reste —S—$R_{13}$ und —OPO($OR_{14}$)$_2$, worin $R_{13}$ Alkyl, Phenyl oder ein Heterocyclus und $R_{14}$ Alkyl oder Phenyl ist, sowie ein Rest der Formel

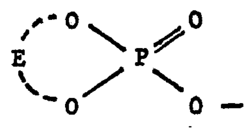

in Betracht, worin E die Ergänzung zu einem Rest mit dem aus dem Phosphoratom, den beiden Sauerstoffatomen und 3 Kohlenstoffatomen bestehenden Ring ist. Als abspaltbares Halogen eignet sich Brom und insbesondere Chlor. Der Alkoxyrest kann 1 bis 4 Kohlenstoffatome enthalten, der Phenoxyrest kann mit Nitro, Carboxyl oder Carboxylester substituiert sein, worin die Alkoholkomponente des Esters 1 bis 4 Kohlenstoffatome enthalten kann. Spezielle Beispiele für Carboxylestersubstituenten sind Methyl-, Aethyl, Propyl- und Butylestergruppen.

Die 5- oder 6-gliedrigen Heterocyclen, die über ein Stickstoffatom an die Kupplungsstelle gebunden sind, sind z.B. Heterocyclen mit einem oder mehreren Stickstoff-, Schwefel und/oder Sauerstoffatomen, die gegebenenfalls mit einem weiteren Ring kondensiert sein können. Beispielsweise genannt seien die Reste von Pyrazol, Imidazol, Triazolen (1, 2, 3 und 1, 2, 4), Tetrazolen, Benztriazol, Pyrimidin, Pyridazin, Thiazol, Oxazol und Oxazin; ferner cyclische Imide. Die genannten Heterocyclen können unsubstituiert oder auch substituiert vorliegen.

Auf folgende Publikationen bezüglich weiterer Einzelheiten über Abgangsgruppen in Zweiäquivalentgelbkupplern wird hingewiesen:

—Halogenatome, wie z.B. in DE—A—2 114 577, FR—A—991 453 und FR—A—869 169 oder US—A—2 728 658 und US—A—3 277 155 beschrieben:

—die Gruppe —OR, wobei R für Alkyl, Aryl, einen heterocyclischen Rest oder steht, wie beispielsweise in GB—A—1 092 506; FR—A—1 411 385 und FR—A—1 385 696 oder in US—A—3 447 928 und US—A—3 408 194 beschrieben;

—die in GB—A—953 454 oder US—A—3 265 506 beschriebene —SR″— Gruppe;

—die 1,2,3-Benztriazolylgruppe der Formel

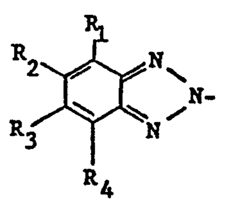

(DE—A—1 800 420)

—die Reste —$SO_3H$ und —SCN (GB—A—638 039; US—A—3 253 924)

—Imidgruppen der Formeln

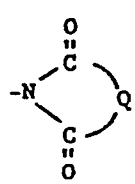 bzw. 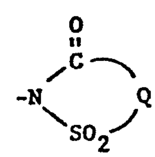

(DE—A—2 163 812, DE—A—2 213 461, DE—A—2 057 941);

—Reste der Formel

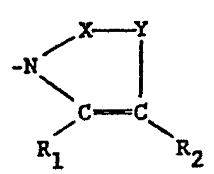

(DE—A—2 329 587).

—Abgangsgruppen der Formel

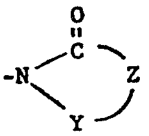

(DE—A—2 433 812)

—1,2,4-Triazolyl- oder 1,2,3-Benzotriazinyl-4-(3)-on-Reste als Abgangsgruppen (DE—A—2 528 638);

—1,2,4-Triazolyl- oder Tetrazolylreste als Abgangsgruppen (DE—A—2 442 703)

—Offenkettige oder cyclische Sulfonamidylreste als Abgangsgruppen (DE—A—2 454 741).

—Abgangsgruppen der Formel

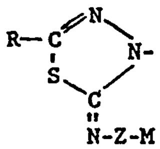

(DE—A—2 716 204)

—Abgangsgruppen der Formel

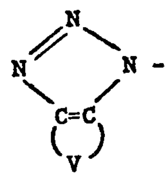

worin V zusammen mit der —C=C— Gruppierung einen aromatischen Ring der Benzolreihe oder einen heterocyclischen Ring mit mindestens einem Stickstoffatom bildet (DE—A—2 414 006).

Die Substituenten $Y_1$ und $Y_2$, die gleich oder voneinander verschieden sein können, können Halogen, z.B. Fluor, Chlor oder Brom, oder Alkyl, Alkoxy oder Alkylmercapto mit vorzugsweise je 1 bis 12 Kohlenstoffatomen, wie beispielsweise Methyl, Aethyl, Propyl, Butyl, Amyl, Hexyl, Octyl, Nonyl, Decyl, Undecyl oder Dodecyl, und die dazugehörigen Isomeren, ferner die analogen Alkoxy oder Alkylmercaptoreste sein. Geeignete Substituenten $Y_1$ und $Y_2$ sind auch —CN, —COOH, Carbalkoxy mit bis zu 12 Kohlenstoffatomen im Alkoxyteil, $—NH_2$, Alkyl-, Phenyl-, Dialkyl- und Diphenylamino, worin Alkyl vorzugsweise 1 bis 5 Kohlenstoffatome enthält oder Acylamino, worin der Acylrest 2 bis 13 Kohlenstoffatome enthält und sich in der Regel von Alkylcarbonsäuren mit der entsprechenden Zahl an Kohlenstoffatomen oder von Benzoesäure ($C_6H_5COOH$) ableitet.

Der Rest $—NHCOR_{15}$ worin $R_{15}$ Alkyl mit bis 12 Kohlenstoffatomen ist und ferner insbesondere Chlor sowie Methyl und Methoxy sind bevorzugte Bedeutungen für $Y_1$ und $Y_2$.

Das Brüchenglied Z, das sich in para-Stellung zu den Substituenten $Y_1$ und $Y_2$ befindet, kann gegebenenfalls substituiertes Alkylen oder Alkenylen; ferner Cycloalkylen oder ein Brückenglied sein, das als verbindende Elemente Amino-, Acyl- und/oder Acylaminofunktionen aufweist, worin Acyl sich von organischen oder anorganischen Sauerstoffsäuren ableitet.

Z ist z.B. geradkettiges Alkylen mit 1 bis 20 Kohlenstoffatomen. Als solche Alkylenreste können also infrage kommen: Methylen, Aethylen, Propylen, Butylen, Pentylen, Hexylen, Heptylen, Octylen, Nonylen, Decylen, Undecylen, Dodecylen, Tridecylen, Tetradecylen, Pentadecylen, Hexadecylen, Heptadecylen, Octadecylen, Nonadecylen, und Eicosylen.

Das geradkettige Alkylenbrückenglied kann ferner mit Alkyl, Hydroxyalkyl, Alkoxyalkyl, Aminoalkyl, Acylaminoalkyl oder Halogenalkyl (Halogen: F, Cl, Br) mit 1 bis 10 Kohlenstoffatomen im Alkylteil und 1 bis 5 Kohlenstoffatomen im Alkoxy- und Acylteil substituiert sein.

Weitere Substituenten können Hydroxyl (OH), Halogen (F, Cl, Br); Alkoxy ($C_1—C_5$); Amino ($—NH_2$), N-Alkyl- und N,N-Dialkylamino, z.B. $CH_3NH—$, $C_5H_{11}NH—$,

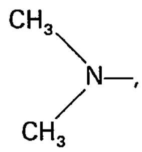,
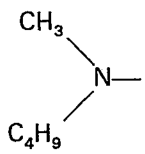

und die entsprechenden Homologen sein.

Als weitere Substituenten am geradkettigen Alklen (Z) seien Mercapto (—SH) und Alkylmercapto ($CH_3S$—, $C_5H_{11}S$—) genannt.

Die genannten Substituenten können einmal oder auch mehrere Male am Alkylenbrückenglied vorhanden sein. Ist Z Alkenylen so kann es 2 bis 20, insbesondere 2 bis 10 Kohlenstoffatome enthalten und ist z.B. Aethenylen, Propenylen, Butenylen, Pentenylen oder Decenylen. Ist Z Cycloalkylen, so kann es 3 bis 12 Kohlenstoffatome aufweisen. Es kommen bevorzugt die dem Cycloalkyl analogen 2-wertigen Reste, wie sie für $R_1$ bis $R_4$ genannt wurden, infrage.

Weitere Brückenglieder (Z) sind:

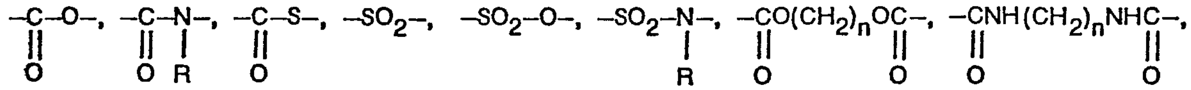

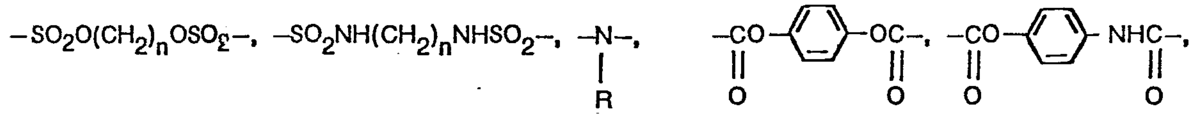

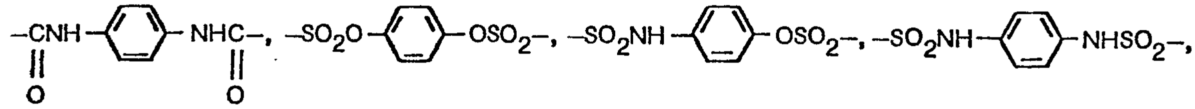

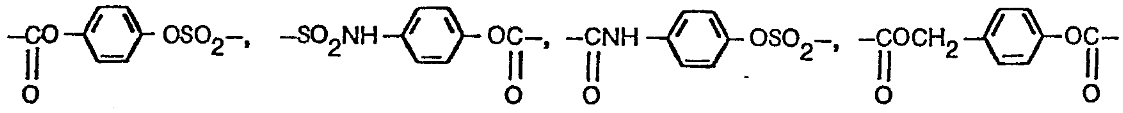 oder

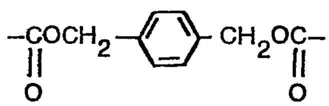

worin R Wasserstoff, Alkyl mit 1 bis 10 Kohlenstoffatomen, gegebenenfalls substituiert mit Halogen, Hydroxyl oder Acyl mit 2 bis 5 Kohlenstoffatomen und n eine ganze Zahl von 2 bis 20 ist.

Ist R Alkyl mit 1 bis 10 Kohlenstoffatomen und kann es z.B. Methyl, Aethyl, Propyl, Butyl, Pentyl, Hexyl, Heptyl, Octyl, Nonyl, Decyl, Undecyl oder Dodecyl oder ein dazugehöriges Isomer (verzweigtes Alkyl) sein. Die Halogen-substituenten an Alkyl sind vorzugsweise Fluor, Chlor und Brom während Acyl z.B. Acetyl, Propionyl, Butanoyl oder Pentanoyl ist.

Die Bindung dieser Brückenglieder an die beiden Phenylringe erfolgt in der für die Brückenglieder angegebenen Schreibweise. Die Links-Rechts-Anordnungen sowohl in den Formeln als auch in den Brückengliedern sind also unverändert zu lassen.

Die Formel (1) umfasst Gelbkuppler der Formel

(2) 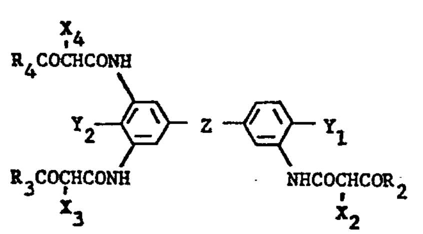 und (3) 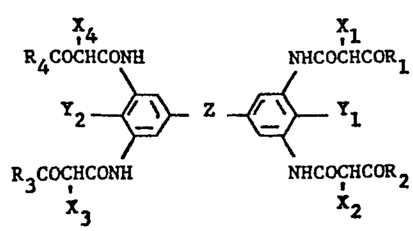

worin Z die angegebene Bedeutung hat, insbesondere aber geradkettiges Alkylen mit 1 bis 20 Kohlenstoffatomen, gegebenenfalls substituiert mit Alkyl, Hydroxyalkyl, Alkoxyalkyl, Aminoalkyl, Acylaminoalkyl oder Halogenalkyl, mit je 1 bis 10 Kohlenstoffatomen im Alkylteil, Hydroxyl, Halogen, Alkoxy mit 1 bis 5 Kohlenstoffatomen, Amino (—$NH_2$), N-Alkylamino und N,N-Dialkylamino mit je 1 bis 5 Kohlenstoffatomen im Alkylteil, Mercapto (—SH) und/oder Alkylmercapto mit 1 bis 5 Kohlenstoffatomen ist sowie ferner Cycloalkylen mit 3 bis 12 Kohlenstoffatomen ist und $R_1$, $R_2$, $R_3$, $R_4$, $X_1$ $X_2$, $X_3$, $X_4$, $Y_1$ und $Y_2$ die angegebenen Bedeutungen haben.

Bevorzugt ist nun solches farbphotographisches Aufzeichnungsmaterial, das als Gelbkuppler Verbindungen der Formel

(4) 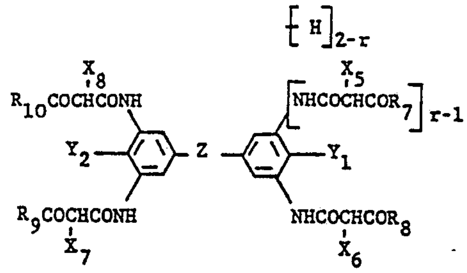

enthält worin

$R_7$, $R_8$, $R_9$ und $R_{10}$ geradkettiges oder verzweigtes Alkyl mit 1 bis 18 Kohlenstoffatomen, Cycloalkyl, Bicycloalkyl oder Tricycloalkyl mit 3 bis 12 Ringkohlenstoffatomen, Phenyl oder Phenyl substituiert mit Halogen, Alkyl oder Alkoxy mit je 1 bis 4 Kohlenstoffatomen oder substituiert mit $—O(CH_2)_rO—$, $—NH_2$, $—SO_2R_{11}$ oder $—NHCOR_{12}$ sind, worin $R_{11}$ Alkyl mit 1 bis 5 Kohlenstoffatomen und $R_{12}$ Alkyl mit 1 bis 5 Kohlenstoffatomen ist,

$X_5$, $X_6$, $X_7$ und $X_8$ Wasserstoff, Halogen, gegebenenfalls substituiertes Alkoxy oder Phenoxy, stickstoffhaltige 5- oder 6-gliedrige Heterocyclen, die über ein Stickstoffatom an die Kupplungsstelle gebunden sind, $—S—R_{13}$ worin $R_{13}$ Alkyl, Phenyl oder ein Heterocyclus ist, $—OPO(OR_{14})_2$, worin $R_{14}$ Alkyl oder Phenyl ist, oder ein Rest der Formel

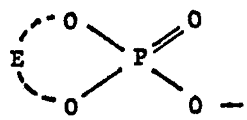

sind, worin E die Ergänzung zu einem Rest mit dem aus dem Phosphoratom, den beiden Sauerstoffatomen und 3 Kohlenstoffatomen bestehenden sechsgliedrigen Ring ist,

$Y_1$ und $Y_2$ Halogen, Alkyl, Alkoxy, Alkylmercapto, —CN, —COOH, Carbalkoxy, $—NH_2$, $—NHR_5$, $—NR_5R_6$, $NHCOR_5$ sind, worin $R_5$ und $R_6$ Alkyl oder Phenyl sind, r 1 oder 2 ist und Z die angegebene Bedeutung hat.

Besonders geeignet sind ferner Aufzeichnungsmaterialien, die Gelbkuppler der Formel

(5) 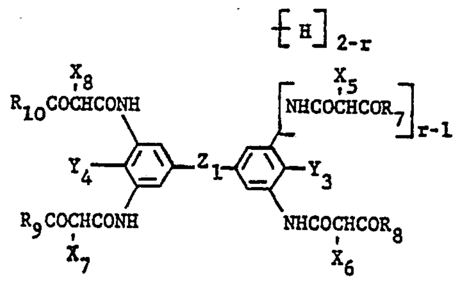

enthalten, worin

$Y_3$ und $Y_4$ Fluor, Chlor, Brom, Alkyl, Alkoxy und Alkylmercapto mit je 1 bis 12 Kohlenstoffatomen, —CN, —COOH, $—COOR_{15}$, $—NH_2$, $—NHR_{16}$, $—NR_{16}R_{17}$ oder $—NHCOR_{15}$ sind, worin $R_{16}$ und $R_{17}$ Alkyl mit 1 bis 5 Kohlenstoffatomen oder Phenyl sind, und $R_{15}$ Alkyl mit 1 bis 12 Kohlenstoffatomen oder Phenyl ist,

$Z_1$ $-(CH_2)_m-$, gegebenenfalls substituiert mit Alkyl, Hydroxyalkyl, Alkoxyalkyl, Aminoalkyl und/oder Halogenalkyl mit je 1 bis 5 Kohlenstoffatomen im Alkyl- und Alkoxy- teil, $Z_1$ ferner

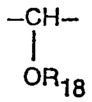, 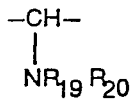, 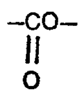, 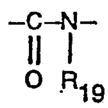, 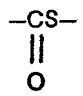, $-SO_2-$, $-SO_2-O-$, 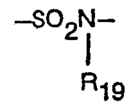,

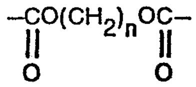, 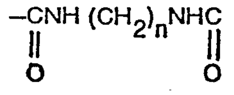, $-SO_2O(CH_2)_nOSO_2-$, $-SO_2NH(CH_2)_nNHSO_2-$, 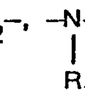

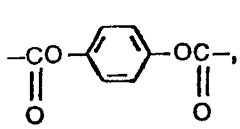, 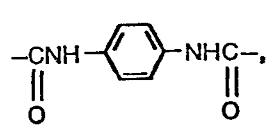, 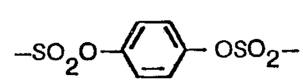, oder

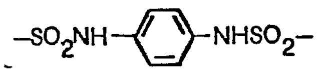 ist,

$R_{18}$ Wasserstoff oder Alkyl mit 1 bis 5 Kohlenstoffatomen ist,

$R_{19}$ und $R_{20}$ Wasserstoff oder Alkyl mit 1 bis 5 Kohlenstoffatomen, gegebenenfalls substituiert mit Halogen, Hydroxyl oder Acyl mit 2 bis 5 Kohlenstoffatomen sind,

m eine ganze Zahl von 1 bis 20,

n eine ganze Zahl von 2 bis 20 ist und

$R_7$, $R_8$, $R_9$, $R_{10}$, $X_5$, $X_6$, $X_7$, $X_8$ und r die in angegebenen Bedeutungen haben.

Bevorzugt sind auch solche farbphotographischen Aufzeichnungsmaterialien, die als Gelbkuppler Verbindungen der Formel

(6) 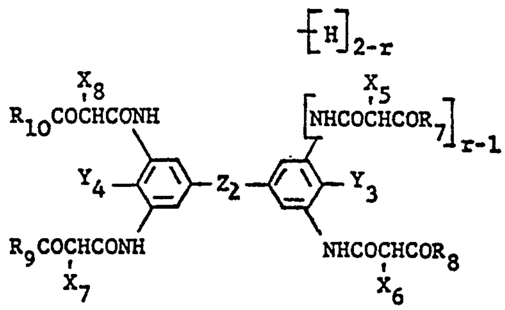

enthalten, worin

$Z_2$ $-(CH_2)_m-$, $-CH(OR_{18})-$, $-CH(NR_{19}R_{20})-$, $-CO-$, $-C(O)-N(R_{19})-$, $-CS-$, $-SO_2-$, $-SO_2-O-$, $-SO_2N(R_{19})-$,

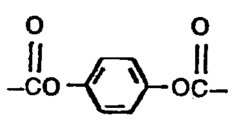 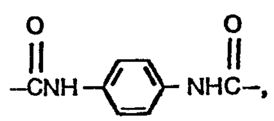, 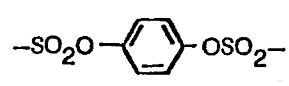 oder

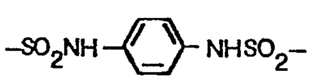 ist und

$R_7$, $R_8$, $R_9$, $R_{10}$, $X_5$, $X_6$, $X_7$, $X_8$, $Y_3$, $Y_4$, $R_{18}$, $R_{19}$, $R_{20}$,
m und r die angegebenen Bedeutungen haben,
oder solche der Formel

(7) 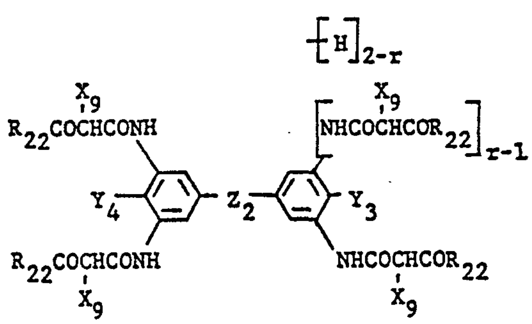

enthalten, worin

$R_{22}$ geradkettiges oder verzweigtes Alkyl mit 1 bis 10 Kohlenstoffatomen, Cyclopentyl, Cyclohexyl, Adamantyl, Phenyl oder Phenyl substituiert mit Chlor oder Brom, Alkyl oder Alkoxy mit je 1 bis 4 Kohlenstoffatomen, $-O-(CH_2)_r-O-$,

$X_9$ Wasserstoff, Chlor oder ein Rest der Formeln

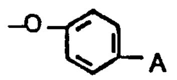 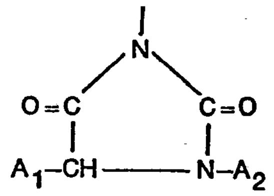 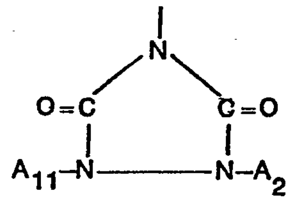 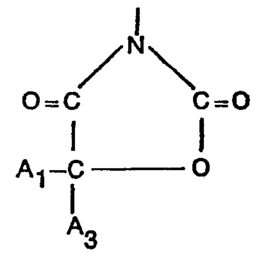

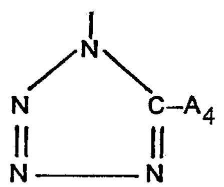 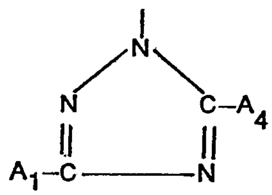 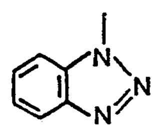 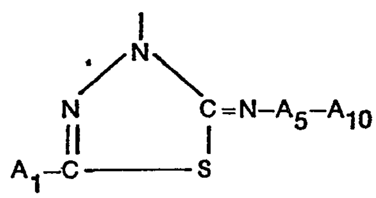

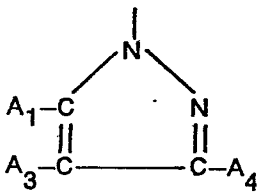 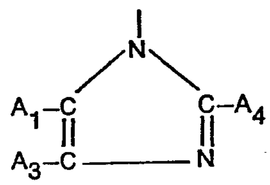 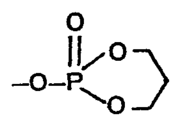 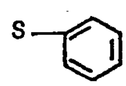 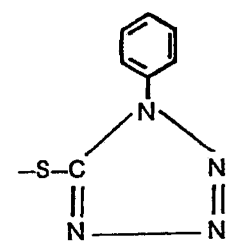

worin A —COOH, —$NO_2$, —$COOR_{21}$, worin $R_{21}$ Alkyl mit 1 bis 4 Kohlenstoffatomen ist, oder der Rest der Formel

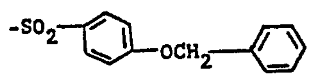 ist,

$A_1$ Wasserstoff, Alkyl mit 1 bis 18 Kohlenstoffatomen, Aralkyl, Aryl, Cycloalkyl mit einem bis vier Cycloalkylringen, Alkoxy mit 1 bis 18 Kohlenstoffatomen, Aryloxy, Alkylmercapto mit 1 bis 18 Kohlenstoffatomen, Arylmercapto, Halogen, Trifluormethyl, Cyan, —$NH_2$, Mono- oder Dialkylamino deren Alkylreste je 1 bis 18 Kohlenstoffatome enthalten,

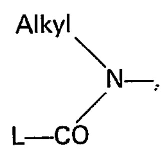

wobei Alkyl 1 bis 5 Kohlenstoffatome enthält,

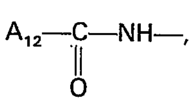, $A_3$—$SO_2$—HN—, 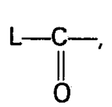, L—$SO_2$—, 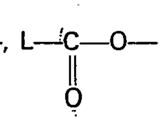 oder 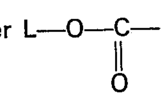,

$A_2$ geradkettiges oder verzweigtes Alkyl mit 1 bis 18 Kohlenstoffatomen, Aralkyl, vorzugsweise Benzyl oder mit Alkyl, Alkoxy, Halogen, $NH_2$, Alkylamino, Dialkylamino, Acylamino, —COOH, Carbalkoxy, Carbonamido, Sulfo, Sulfonamido oder Alkylmercapto substituiertes Phenyl,

$A_3$ unverzweigtes oder verzweigtes Alkyl mit 1 bis 18 Kohlenstoffatomen, gegebenenfalls substituiert mit Halogen, Nitro, Cyan, Alkoxy oder primären, sekundären oder tertiären Aminogruppen Aralkyl, Cycloalkyl mit einem bis vier Cycloalkylringen;

Aryl, gegebenfalls substituiert mit Alkyl oder Alkoxy mit je 1 bis 4 Kohlenstoffatomen, Halogen, Acylamino.

—$SO_3H$, —COOH, Sulfonsäureamid oder Carbonsäureamid, N- oder N,N-substituiertes Sulfonsäure- oder Carbonsäureamid, Carbonsäureester, Hydroxyl, Nitro, primärem, sekundärem oder tertiärem Amin, Mercapto, Alkylmercapto, —$SO_2$—L— oder —CO—L; Pyridyl, Furyl, Thienyl, Perfluoroalkyl, Acyl,

Dialkylamino mit je 1 bis 5 Kohlenstoffatomen im Alkylteil, Alkoxy mit 1 bis 18 Kohlenstoffatomen oder Phenoxy,

$A_4$ Wasserstoff, gegebenenfalls substituiertes Alkyl mit 1 bis 18 Kohlenstoffatomen, Cycloalkyl, Cycloalkenyl, Alkenyl, Aryl, Aralkyl, ein heterocyclischer Rest, Alkoxy, Aryloxy, Alkylmercapto, gegebenenfalls durch Alkyl, Aryl oder Acyl substituiertes Amino, Alkylsulfonyl, Arylsulfonyl, Acyloxy, Aminosulfonyl, Carbonsäuremaid, Sulfonsäureamid, Carbonsäurealkylester, Nitro, Cyan, Halogen, gegebenenfalls substituiertes Ureido oder gegebenenfalls substituiertes Aminosulfonylamino und

$A_5$ —CO— oder —$SO_2$— ist;

$A_{10}$ die Bedeutung von $A_3$ außer —$SO_2L$ oder —COL hat und ausserdem Wasserstoff ist, sofern $A_5$ —CO— ist,

$A_{11}$ Alkyl mit 1 bis 18 Kohlenstoffatomen ist, gegebenenfalls substituiert mit Halogen, Amino, Cyano oder Alkoxy, Cycloalkyl, Aryl oder Aralkyl

$A_{12}$ Wasserstoff ist oder die Bedeutung von $A_3$ hat,

L Alkyl mit 1 bis 18 Kohlenstoffatomen, gegebenenfalls substituiert mit Halogen, Amino, Cyano oder Alkoxy; Cycloalkyl, Aryl, Pyridyl, Pyrimidyl, Furyl oder Thienyl ist und $Y_3$, $Y_4$, $Z_2$ und r die angegebenen bedeutungen haben.

Besonders bevorzugt sind schliesslich solche farbphotographischen Materialien, die die Gelbkuppler der Formel

(8) 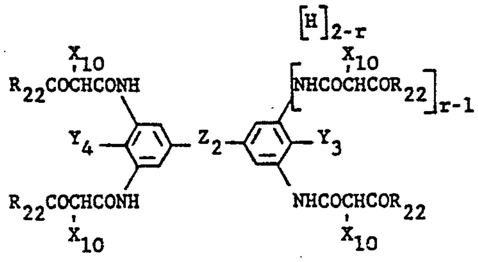

enthalten, worin

$X_{10}$ Wasserstoff, Chlor oder ein Rest der Formeln

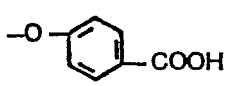
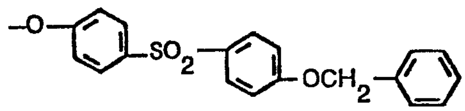
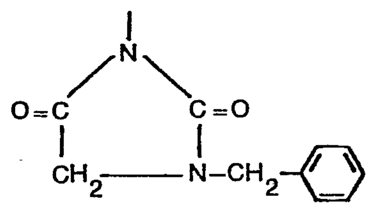
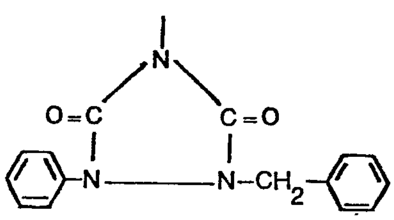
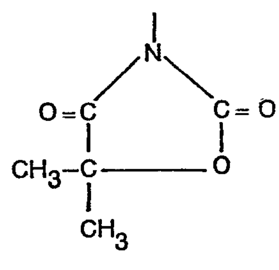
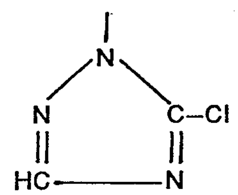
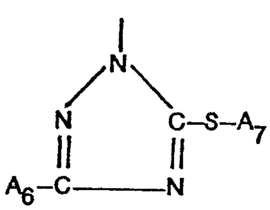
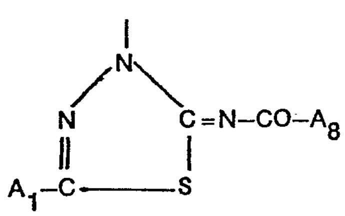
oder
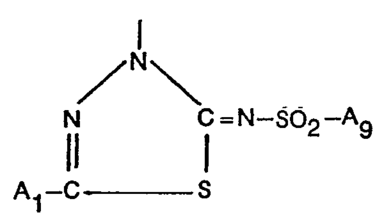
ist,

worin $A_6$ Wasserstoff oder Alkyl mit 1 bis 4 Kohlenstoffatomen, $A_7$ Alkyl mit 1 bis 12 Kohlenstoffatomen, $A_8$ geradkettiges oder verzweigtes Alkyl mit 1 bis 18 Kohlenstoffatomen,

$-CH_2Cl$ $-CCl_3$ 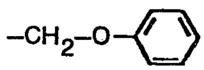 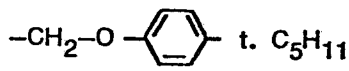 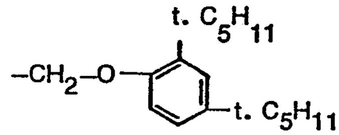

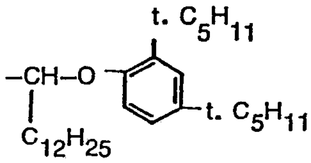 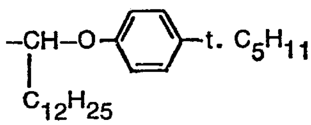 $-CH_2-O-C_pH_{2p+1}$

Aralkyl, Cycloalkyl, Phenyl oder Phenyl substituiert mit

$-C_qH_{2q+1}$ $-OC_qH_{2q+1}$ $-Cl$ $-Br$ $-NHOCC_pH_{2p+1}$ 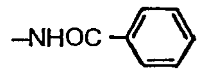 $-SO_2NH_2$

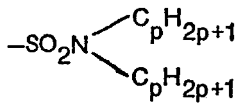 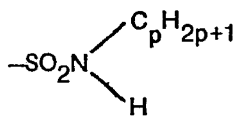 $-CONH_2$ 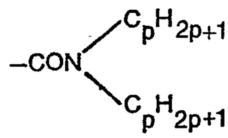 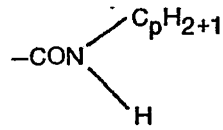

$-SO_2CH_3$ 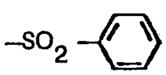 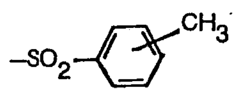 $-COOH$ 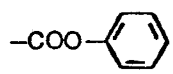 $-COO-C_pH_{2p+1}$

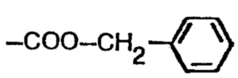 $-OH$ $-NH_2$ 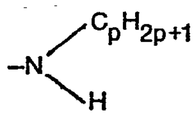 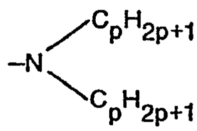 $-SC_pH_{2p+1}$

$-SO_3H$ $-COC_pH_{2p+1}$ oder 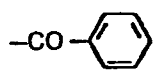

Pyridyl, Furyl, Thienyl, $-C_pF_{2p+1}$ $-COC_pH_{2p+1}$ 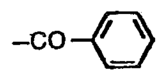 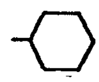 Norbornyl,

Adamantyl, 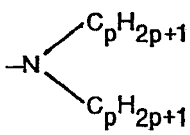 $-O-C_pH_{2p+1}$ oder 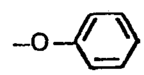

P eine Zahl von 1 bis 5, q eine Zahl von 1 bis 4 und $A_9$ $-CH_3$ 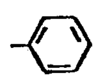 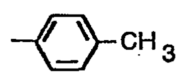

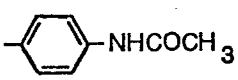 ist,

$R_{22}$ geradkettiges oder verzweigtes Alkyl mit 1 bis 10 Kohlenstoffatomen, Cyclopentyl, Cyclohexyl, Adamantyl, Phenyl oder Phenyl substituiert mit Chlor oder Brom, Alkyl oder Alkoxy mit je 1 bis 4 Kohlenstoffatomen, $-O-(CH_2)_r-O-$, $Y_3$, $Y_4$, $Z_2$ und r die angegebenen Bedeutungen haben.

Besonders geeignete Gelbkuppler der Formel (8) sind solche der Formel

(9) 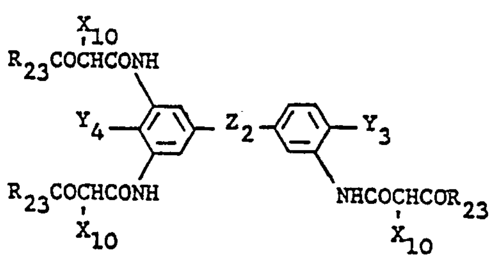 (10) und 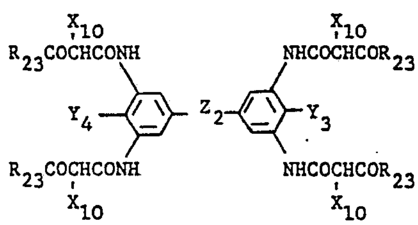

worin $R_{23}$ tert. Alkyl mit 4 bis 8 Kohlenstoffatmen ist und $X_{10}$, $Y_3$, $Y_4$ und $Z_2$ die angegebenen Bedeutungen haben.

Insbesondere ist $R_{23}$ tert.-Butyl, 1,1,3,3-Tetramethylbutyl, 1-Methyl-1-äthylpentyl oder 1,1-Dimethylpropyl. Bevorzugt bedeuten $Y_3$ und $Y_4$ Chlor, Alkoxy oder Alkyl mit 1 bis 5 Kohlenstoffatomen (insbesondere Methyl oder Methoxy). Die besonders bevorzugte Bedeutung von $Z_2$ ist —COO—, —$SO_2$NH—, —$SO_2$— oder insbesondere —CONH—.

Die Herstellung der Gelbkuppler der Formel (1) kann z.B. so erfolgen, dass man Tri- oder Tetranitroverbindungen der Formel

(11) 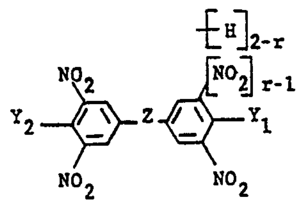

worin $Y_1$, $Y_2$, Z und r die angegebenen Bedeutungen haben, zu den entsprechenden Aminoverbindungen (Tri- und Tetramine) reduziert und diese dann mit geeigneten Acylverbindungen umsetzt.

Die Verbindungen der Formel (11) sind zum Teil aus der Literatur bekannt oder werden nach allgemein bekannten chemischen Methoden hergestellt, z.B. durch Reaktionen, wie sie in den folgenden Gleichungen angegeben werden:

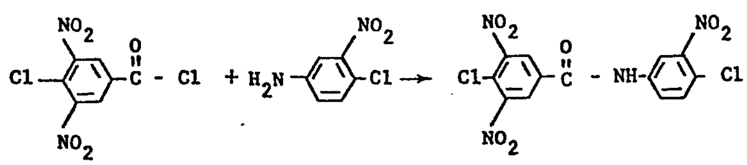

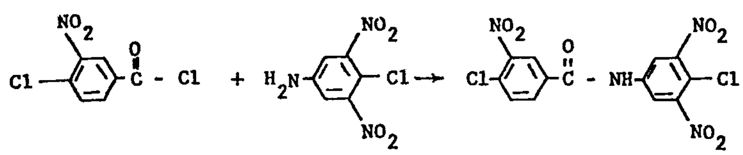

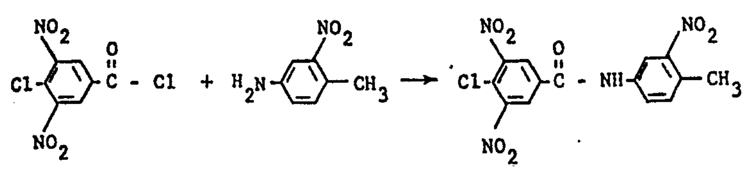

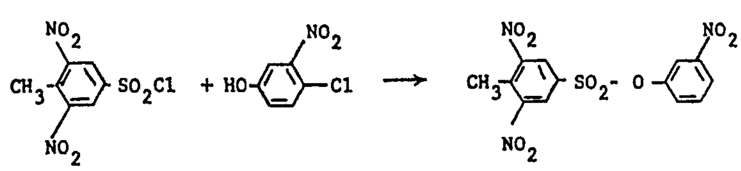

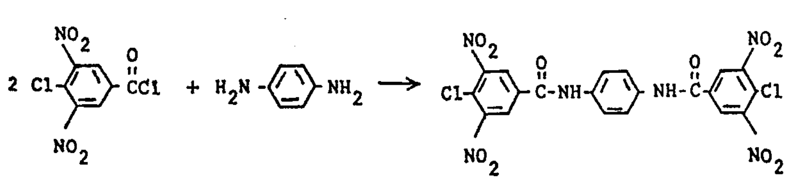

Als Ausgangsprodukte benötigte Dinitrobenzoesäuren, Dinitrosulfonsäuren, Dinitroamine oder Dinitrophenole der Formeln

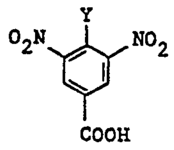

(12)

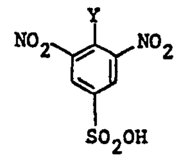

(13)

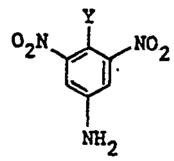

(14)

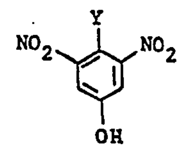

(15)

sind aus der Literatur bekannt. Sie lassen sich nach verschiedenen Methoden herstellen, wie sie z.B. in Org. Synth. Coll. Vol. *4* 364; Ann. *366*, 95; Ann. *274*, 349; J. Am. Chem. Soc. *49*, 497, (1927); Ber. *42*, 1729; Ber. *10*, 1696 und Ber. *58*, 1221 beschrieben sind.

Tri- bzw. Tetramine der Formel

(16)

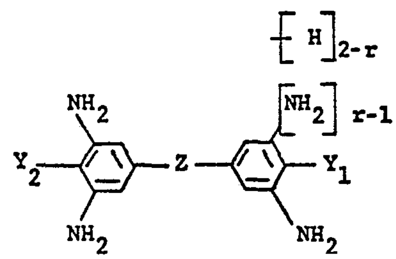

worin $Y_1$, $Y_2$, Z und r die angegebenen Bedeutungen haben, werden mit mindestens 3 oder 4 Mol eines Esters der Formel

(17)

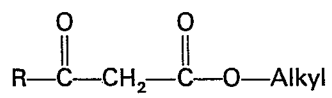

umgesetzt. R hat die für $R_1$ bis $R_4$ angegebenen Bedeutungen. Ester der Formel (17) sind aus der Literatur bekannt (z.B. US—A—3 245 506 und US—E—27 848, Org. Reactions, *1*, 266 ff — Wiley New York — und J.A.C.S. *70*, 497 (1948)).

Man erhält sie beispielsweise durch Kondensation eines Säurechlorides der Formel

(18)

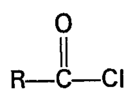

mit der Natriumverbindung eines Acetessigsäurealkylesters anschliessende Spaltung mit einer Base (vgl. z.B. DE—A—25 03 099 und DE—A—2 114 577, Org. Synth. Coll, Vol. II, 266; J. Am. Chem. Soc. *67*, 2197 (1945)).

Aus der Reaktion der Ketoester der Formel (17) mit den Tri- oder Tetramin der Formel (16) erhält man die Gelbkuppler der Formel

(19)

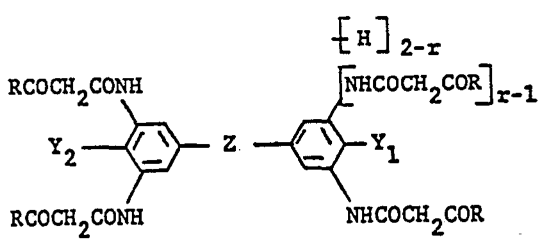

Die nach dem beschriebenen Herstellungsverfahren erhaltenen 3 oder 4 mal 4-Aequivalentkuppler der Formel (19) können durch Ersatz eines Wasserstoffatoms der $—CH_2—$ Gruppe durch ein Halogenatom in an sich bekannter Weise umgewandelt werden. (vgl. z.B. DE—A—2 263 875, DE—A—2 402 220 und DE—A—2 329 587; US —A—3 265 506).

Diese 3 oder 4 mal 2-Aequivalentkuppler entsprechen der Formel

(20) 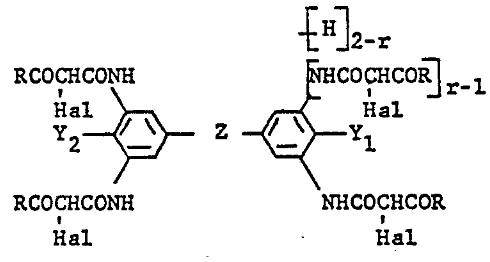

worin Hal Chlor oder Brom bedeutet und die übrigen Symbole die angegebenen Bedeutungen haben.

Die Zwei äquivalentkuppler der Formel (20) können vorzugsweise mit Salzen der Formel

$$X^{\ominus}M^{\oplus}$$

weiter umgesetzt werden. X ist ein während der Kupplungsreaktion abspaltbarer Rest ($X_1$ bis $X_4$) und $M^{\oplus}$ ist ein Kation, z.B. Na, K oder Ag. Die Gelbkuppler der Formel (1) bis (10) können in bekannter Weise in eine Gelatine oder auch ein anderes Bindemittel enthaltende Silberhalogenidemulsion eingearbeitet werden.

Sie können allein oder auch im Gemisch, gegebenenfalls sogar im Gemisch mit anderen Kupplern, verwendet werden. Ihre Einsatzmenge beträgt zwischen 50—300 g pro Mol Silberhalogenid.

Die zur Herstellung des erfindungsgemässen photographischen Materials verwendeten Silberhalogenidemulsionen können z.B. Silberbromid, Silberjodid, Silberchlorid, Silberchlorbromid, Silberjodbromid und Silberchlorjodbromid enthalten. Gute Ergebnisse werden auch bei Verwendung mindestens einer photographischem·Emulsionsschicht erhalten, die Silberchlorjodid,·Silberjodbromid oder Silberchlorjodbromid mit einem Jodgehalt von 1 bis 20 Mol-% enthält.

Es kann sich hierbei um übliche negative als auch um direkt positive Emulsionen handeln. Die Emulsionen können die üblichen Zusätze, wie z.B. Härter, Sensibilisatoren, Stabilisatoren, Netzmittel und Antischleiermittel enthalten.

Als Bindemittel für die photographischen Schichten wird vorzugsweise Gelatine verwendet. Diese kann jedoch ganz oder teilweise durch andere natürliche oder synthetische Bindemittel ersetzt werden. Als natürliche Bindemittel sind z.B. Alginsäure ersetzt werden. Als natürliche Bindemittel sind z.B. Alginsäure und deren Derivate wie Salze, Ester oder Amide, Cellulosederivate wie Carboxymethylcellulose, Alkylcellulose wie Hydroxyäthylcellulose, oder Stärke, und deren Derivate wie Aether oder Ester geeignet. Als synthetische Bindemittel können beispielsweise Acrylharze, Polyvinylalkohol, teilweise verseiftes Polyvinylacetat oder Polyvinylpyrrolidon infrage kommen. Schichtträger für das erfindungs gemässe photographische Material sind die für diesen Zweck üblichen und geeigenten Folien, z.B. aus Cellulosenitrat, Celluloseacetat, wie Cellulosetriacetat, Polystyrol, Polyestern, wie Polyäthylenterephthalat, Polyolefinen wie Polyäthylen oder Polypropylen, ferner gegebenenfalls beschichtete Papiere, wie z.B. polyäthylenbeschichtete Papiere, sowie Glas.

Entsprechende Anwendungsmöglichkeiten sind beispielsweise in US—A—2 304 939, US—A—2 304 940, US—A—2 322 027, US—A—2 284, 897, US—A—2 801 170, US—A—2 801 171, US—A—2 749 360 und US—A—2 825 382 beschrieben. Gegebenenfalls können die Kuppler auch im Entwicklungsbad eingesetzt werden.

Die photographischen Entwicklungsbäder enthalten im allgemeinen ein aromatisches, primäres Amin als Entwicklersubstanz, vorzugsweise ein p-Phenyendiaminderivat, wie z.B. 4-Amino-N,N-dimethylanilin, 4-Amino-N,N-diäthylanilin, 4-Amino-3-methyl-N-methyl(äthyl)-N-(β-methylsulfonamidoäthyl)anilin oder 4-Amino-3-methyl-N-äthyl-N-(β-hydroxyäthyl)anilin, ferner Monomethyl-p-phenylendiamin, 2-Amino-5-diäminotoluol, N-Butyl-N-ω-sulfobutyl-p-phenylendiamin, N-Aethyl-N-β-hydroxyäthyl-p-phenylendiamin, N,N-bis-(β-Hydroxyäthyl)-p-phenylendiamin oder 2-Amino-5-(N-äthyl-N-β-hydroxyäthylamino)-toluol.

Die erfindungsgemäss verwendeten Kuppler haben eine hohe Kupplungsreaktivität gegenüber dem Oxydationsprodukt des aromatischen primären Amins (Entwickler), sodass die Entwicklung der Silberhalogenidemulsion und die Bildung des Farbstoffes rasch ablaufen.

Man erzielt ferner eine hervorragende Reaktivität und eine gute Farbdichte bei einer gleichzeitig deutlich herabgesetzen molaren Einsatzmenge der erfindungsgemässen Kuppler im Vergleich mit bekannten Kupplern. Da gleichzeitig die Lösungsmittelmenge für den Kuppler verringert werden kann, ist es möglich, die Gesamtschichtdicke der Emulsionsschicht zu verringern.

Verwendet man Zweiäquivalentkuppler, so kann man ausserdem die Silberhalogenidmenge verringern. Alle Massnahmen setzen die Herstellungskosten für das photographische Material herab.

Die für Blaulicht empfindliche Schicht kann dünner gehalten werden, wodurch die Schärfe und die Auflösung des erhaltenen Farbbildes verbessert wird.

Das mit den erfindungsgemäss eingesetzten Gelbkupplern erhaltene farbphotographische Bild zeigt ausgezeichnete spektrale Absorptionseigenschaften sowie eine gute Licht-, Wärme- und Feuchtigkeitsbeständigkeit und damit eine genügend grosse Stabilität, um es über längere Zeit ohne

irgendwelche Beeinträchtigungen lagern zu können. In den nachfolgenden Beispielen beziehen sich Teile und Prozente auf das Gewicht.

Beispiel 1

Eine Lösung von 25,6 g N-[4-Chlor-3,5-diaminobenzoyl]-4-chlor-3-aminoanilin und 53 g Pivaloylessigsäuremethylester in 2 Litern Xylol werden während 5 Stunden auf 140°C erhitzt. Der entstehende Alkohol wird dabei laufend durch Destillation entfernt. Nach Beendigung der Reaktion wird das Reaktionsgemisch in Eis gekühlt, der entstandene Niederschlag abfiltriert und mit Hexan nachgewaschen. Man erhält 51,4 g der Verbindung der Formel

(101)

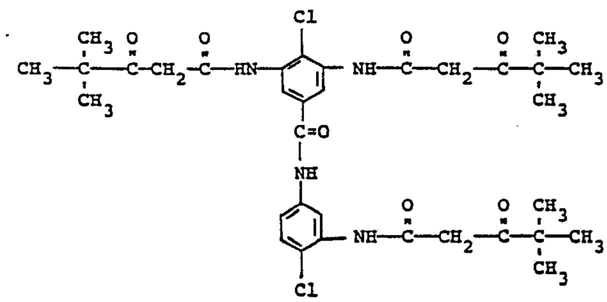

Schmelzpunkt: 165 bis 168°C.

Auf analoge Weise lassen sich auch die übrigen in Tabelle I angegebenen Gelbkuppler herstellen.

TABELLE I

Gelbkuppler der Formel

(100)

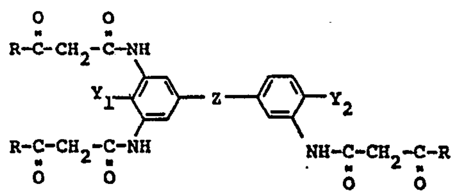

| Nr. | R | Z | $Y_1$ | $Y_2$ | Schmelzpunkt °C | $D_{max}$ |
|---|---|---|---|---|---|---|
| 101 | t-Butyl | –CONH– | Cl | Cl | 165 – 168 | 1,05 |
| 102 | t-Butyl | $-SO_2-NH-$ | $CH_3$ | Cl | 142 – 146 | 1,10 |
| 103 | t-Butyl | $-SO_2-$ | Cl | Cl | 100 – 102 | 1,26 |
| 104 | t-Butyl | –CONH– | Cl | $CH_3$ | 138 – 140 | 1,10 |

Beispiel 2

Zu einer Lösung von 17,5 g des Kupplers der Formel (101) in 1,5 Litern Tetrachlorkohlenstoff werden 13 g Brom langsam bei 0°C zugetropft. Anschliessend wird das Eisbad entfernt und das Gemisch solange gerührt, bis Raumtemperatur erreicht wird. Man wäscht die Lösung mit Wasser neutral und trocknet die organische Phase über Natriumsulfat. Nach Entfernen des Lösungsmittels erhält man einen festen Rückstand, der aus einem Chloroform/Hexangemisch umkristallisiert wird. Man erhält 24,5 g der Verbindung der Formel

(201)

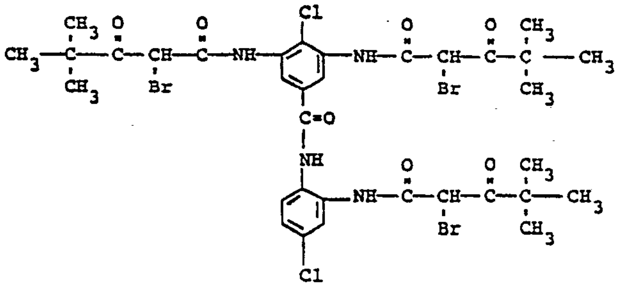

mit einem Schmelzpunkt von 140 bis 142°C (Kuppler Nr. 201 in Tabelle II)

Auf gleiche Weise könnendie übrigen Gelbkuppler der Tabellen II und IV, bei denen X gleich Br ist, hergestellt werden.

Beispiel 3

Ein Gemisch aus 5,9 g des Kupplers der Formel (201), 1,10 g Kaliumhydroxyd und 4,76 g 2-Pivaloylamino-5-tert.-butyl-1,3,4-thiadiazol in 300 ml Dimethylformamid werden bei Raumtemperatur während 10 Stunden gerührt. Das Gemisch wird auf Wasser gegossen, der entstandene Niederschlag abfiltriert und mehrfach aus einem Methanol/Wassergemisch umkristallisiert. Man erhält 2,9 g der Verbindung der Formel

(202)

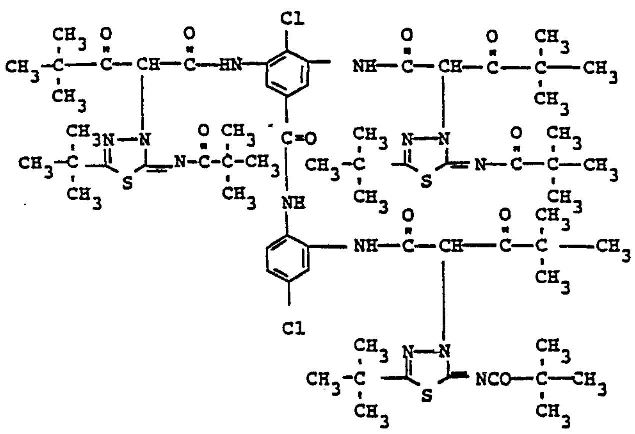

mit einem Schmelzpunkt von 163 bis 166°C. Analog lassen sich auch die übrigen Gelbkuppler der Tabelle II herstellen.

TABELLE II

Gelbkuppler der Formel

(200) 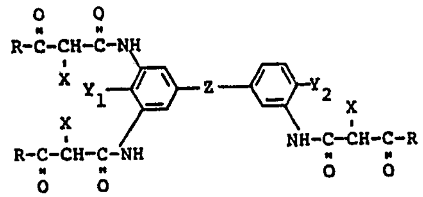

| Nr. | R | Z | $Y_1$ | $Y_2$ | X | Schmelzpunkt | $D_{max}$ |
|---|---|---|---|---|---|---|---|
| 201 | t-Butyl | –CONH– | Cl | Cl | Br | 140 – 142 | 1,29 |
| 202 | t-Butyl | –CONH– | Cl | Cl | 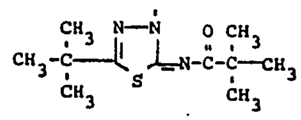 | 162 – 163 | 1,41 |
| 203 | t-Butyl | –CONH– | Cl | Cl | 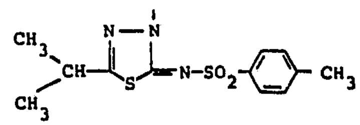 | 160 – 165 | 1,53 |
| 204 | t-Butyl | –CONH– | Cl | Cl | 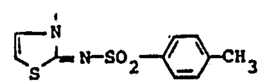 | 192 – 195 | 1,28 |
| 205 | t-Butyl | –CONH– | Cl | Cl | 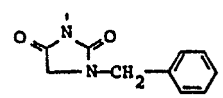 | 162 – 165 | 1,15 |
| 206 | t-Butyl | –CONH– | Cl | Cl | 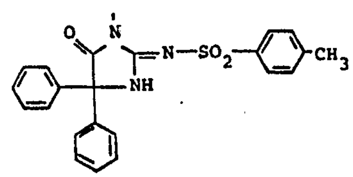 | 198 – 200 | 1,28 |

TABELLE II (Fortsetzung)

| Nr. | R | Z | $Y_1$ | $Y_2$ | X | Schmelzpunkt °C | $D_{max}$ |
|---|---|---|---|---|---|---|---|
| 207 | t-Butyl | $-SO_2-NH-$ | $CH_3-$ | Cl | Cl | 125 – 126 | 1,28 |
| 208 | t-Butyl | $-CONH-$ | Cl | Cl | 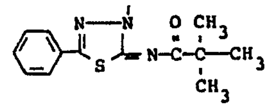 | 179 – 180 | 1,23 |
| 209 | t-Butyl | $-CONH-$ | Cl | Cl | 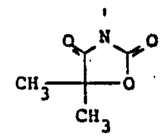 | 196 – 197 | 1,35 |
| 210 | t-Butyl | $-SO_2NH-$ | $CH_3-$ | Cl | 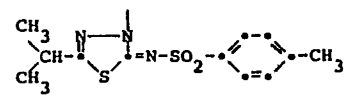 | 170 – 172 | 1,34 |
| 211 | t-Butyl | $-SO_2-$ | Cl | Cl | Cl | 130 – 132 | 1,38 |
| 212 | t-Butyl | $-SO_2-$ | Cl | Cl | 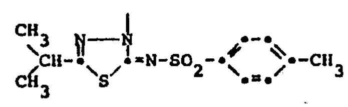 | 172 – 174 | 1,55 |
| 213 | t-Butyl | $-SO_2-$ | Cl | Cl | 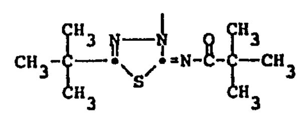 | 182 – 185 | 1,28 |
| 214 | t-Butyl | $-CONH-$ | Cl | $CH_3-$ | Cl | 116 – 118 | 1,10 |
| 215 | t-Butyl | $-CONH-$ | Cl | $CH_3-$ | 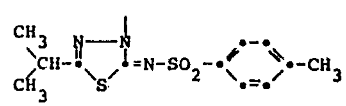 | 157 – 160 | 1,23 |
| 216 | t-Butyl | $-CONH-$ | Cl | $CH_3-$ | 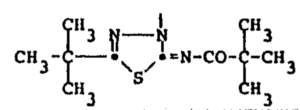 | 162 – 165 | 1,26 |

TABELLE II (Fortsetzung)

| Nr. | R | Z | $Y_1$ | $Y_2$ | X | Schmp. °C | $D_{max}$ |
|---|---|---|---|---|---|---|---|
| 217 | t-Butyl | —CONH— | Cl | $CH_3O-$ | 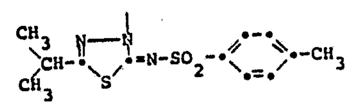 | 150 – 152 | 1,20 |
| 218 | t-Butyl | —CONH— | Cl | Cl | 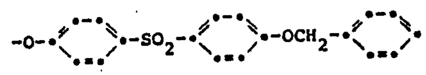 | 136 – 137 | 1,20 |

Beispiel 4

Eine Lösung von 13 g N-[4-Chloro-3,5-diaminobenzoyl]-4-chlor-3,5-diaminoanilin und 44 g Pivaloylessigsäuremethylester in 2 Litern Xylol werden unter Abdestillieren des entstehenden Methanols während 2 Stunden am Rückfluss erhitzt. Nach Beendigung der Reaktion wird das Xylol abgedampft und der Rückstand aus einem Chloroform/Hexangemisch und anschliessend aus Methanol umkristallisiert. man erhält die Verbindung der Formel

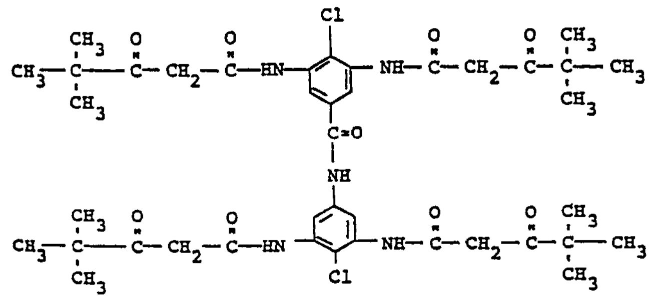

mit einem Schmelzpunkt von 150 bis 155°C. Ausbeute: 68% Analog lassen sich auch die übrigen Gelbkuppler der Tabelle III herstellen.

TABELLE III

Gelbkuppler der Formel

(300)

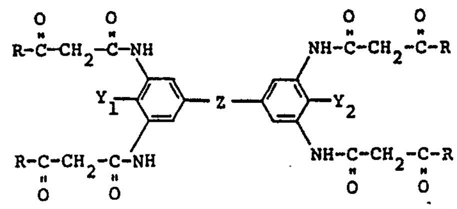

| Nr. | R | Z | $Y_1$ | $Y_2$ | Schmelzpunkt °C | $D_{max}$ |
|---|---|---|---|---|---|---|
| 301 | t-Butyl | –CONH– | Cl | Cl | 150 – 155 | 1,18 |
| 302 | t-Butyl | $-SO_2-$ | Cl | Cl | 154 – 156 | 1,16 |

Beispiel 5

Ein Gemisch aus 1,28 g eines Kupplers der Formel

(401)

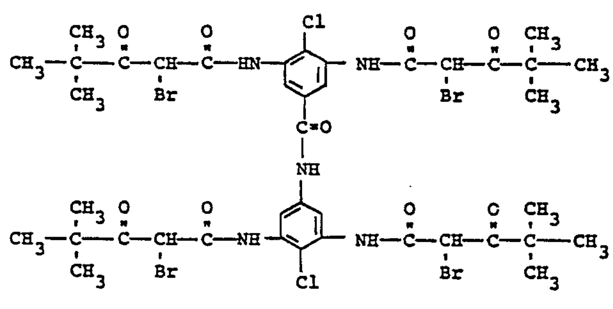

(hergestellt analog dem Verfahren von Beispiel 2) und 1,53 g des Kaliumsalzes von 2-p-Toluolsulfonylamino-5-isopropyl-1,3,4-thiadiazol in 400 ml Acetonitril wird bei Raumtemperatur während 6 Stunden gerührt. Das Gemisch wird auf angesäuertes Wasser gegossen, der entstandene Niederschlag wird abfiltriert und zweimal aus einem Chloroform/Hexangemisch umkristallisiert.

Man erhält die Verbindung (402) der Tabelle IV als dünnschicht-chromatographisch einheitliches Produkt. Schmelzpunkt: 168 bis 170°C. Ausbeute: 95%.

TABELLE IV

Gelbkuppler der Formel

(400) 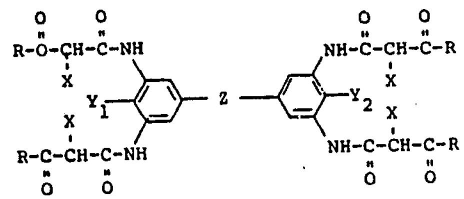

| Nr. | R | Z | $Y_1$ | $Y_2$ | X | Schmp. °C | $D_{max}$ |
|---|---|---|---|---|---|---|---|
| 401 | t-Butyl | –CONH– | Cl | Cl | Br | 139–144 | 1,29 |
| 402 | t-Butyl | –CONH– | Cl | Cl | 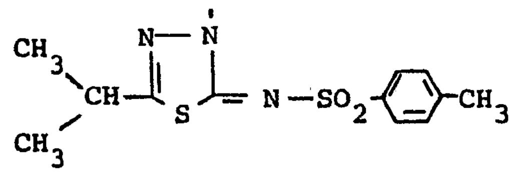 | 165–170 | 1,62 |
| 403 | t-Butyl | –CONH– | Cl | Cl | 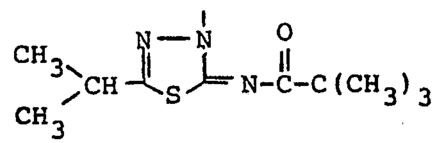 | 175–177 | 1,30 |
| 404 | t-Butyl | –CONH– | Cl | Cl | Cl | 150–153 | 1,10 |

Anwendungsbeispiel

Beispiel 6

*Kuppler-Emulsion*

0,033 mMol Gelbkuppler der Formel (203) werden in 2,0 ml Trikresylphosphat-Methylenchlorid (1:9) gelöst. Man dampft das Methylenchlorid ab, gibt 6,6 ml 6%ige Gelatinelösung, 1,2 ml Wasser und 2,0 ml einer 8%igen, wässrigen Lösung von Natrium-isopropylnaphthalinsulfonat hinzu, stellt das Gemisch auf einen pH-Wert von 6,5 ein und emulgiert mit Hilfe eines Ultraschallgerätes während 5 Minuten mit einer Leistung von 100 Watt.

*Guss*

2,5 ml frisch beschallte Kuppler-Emulsion, 1,6 ml Silberbromid-Emulsion vom pH 6,5 mit einem Gehalt von 0,7% Silber und 6,0% Gelatine, 1,0 ml, 1%ige wässrige Lösung des Härters der Formel

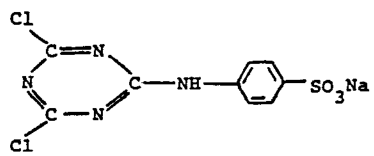

und 5,0 ml Wasser werden miteinander vermischt und bei 40°C auf eine substrierte Glasplatte von 13 cm · 18 cm vergossen.

Nach dem Erstarren bei 10°C wird die Platte in einem Trockenschrank mit, Umluft bei Raumtemperatur getrocknet.

*Photographische Belichtung und Verarbeitung*

Ein auf 4,0 cm · 6,5 cm geschnittener Streifen wird unter einem Stufenkeil während 6 Sekunden mit 500 Lux/cm² belichtet und anschliessend bei 24°C folgendermassen behandelt:

| | Minuten |
|---|---|
| 1. Farbentwicklung | 5 |
| 2. Wässern | 5 |
| 3. Erste Fixierung | 2 |
| 4. Wässern | 2 |
| 5. Silberbleichen | 4 |
| 6. Wässern | 2 |
| 7. Zweite Fixierung | 4 |
| 8. Wässern | 10 |
| 9. Trocknen | 10 |

Die Verarbeitungslösungen besitzen folgende Zusammensetzung:

I. Farbentwicklungslösung (pH = 10,7)

| | |
|---|---|
| 4-Amino-3-methyl-N-äthyl-N-β-(methylsulfonamido)äthyl-anilin; 1 1/2 $H_2SO_4 \cdot H_2O$ | 10 mMol |
| wasserfreies Natriumsulfit | 2,0 g |
| Kaliumbromid | 0,5 g |
| Kaliumcarbonat | 40,0 g |
| Benzylalkohol | 10,0 g |
| Wasser | auf 1000 ml |

| | | |
|---|---|---|
| II. | Fixierlösung (pH = 4,5) | |
| | Natriumthiosulfat · 6 $H_2O$ | 80,0 g |
| | wasserfreies Natriumsulfit | 5,0 g |
| | Natriumborat (Borax) | 6,0 g |
| | Kaliumalaun | 7,0 g |
| | Essigsäure | 4,0 g |
| | Wasser | auf 1000 ml |
| III. | Silberbleichbad (pH = 7,2) | |
| | Kaliumhexacyanoferrat (III) | 100,0 g |
| | Borsäure | 10,0 g |
| | Natriumborat (Borax) | 5,0 g |
| | Wasser | auf 1000 ml |

Man erhält einen klaren, scharfen Gelbkeil mit einem Absorptionsmaximum bei 446 nm und einer maximalen Farbdichte von 1,53.

In gleicher Weise können auch mit den anderen in den Beispielen 1 bis 5 einschliesslich der Tabellen beschriebenen Gelbkupplern photographische Materialien hergestellt und verarbeitet werden. Man erhält dann die in den Tabellen angegebenen $D_{max}$-Werte.

**Patentansprüche**

1. Farbphotographisches Aufzeichnungsmaterial, das in mindestens einer Silberhalogenidemulsionsschicht mindestens einen Gelbkuppler mit mehreren während einer Kupplungsreaktion abspaltbaren Gruppenenthält, dadurch gekennzeichnet, dass der Gelbkuppler der Formel Formel

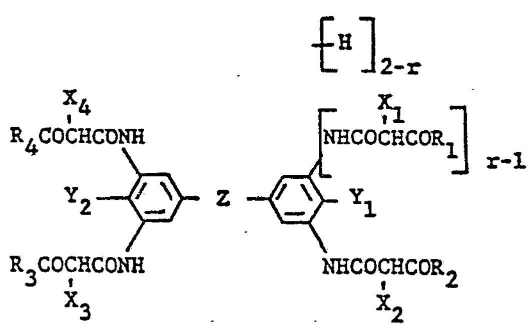

entspricht, worin

$R_1$, $R_2$, $R_3$ und $R_4$ Alkyl, Cycloalkyl oder Aryl,

$X_1$, $X_2$, $X_3$ und $X_4$ während einer Kupplungsreaktion abspaltbare Reste, und

$Y_1$ und $Y_2$ Halogen, Alkyl, Alkoxy, Alkylmercapto, —CN, —COOH, Carbalkoxy, —$NH_2$, —$NHR_5$, —$NR_5R_6$ oder —$NHCOR_5$ sind, worin $R_5$ und $R_6$ Alkyl oder Phenyl sind, und

Z ein geradkettiges Alkylen mit 1 bis 20 Kohlenstoffatomen, gegebenenfalls substituiert mit Alkyl, Hydroxyalkyl, Alkoxyalkyl, Aminoalkyl, Acylaminoalkyl oder Halogenalkyl mit je 1 bis 10 Kohlenstoffatomen im Alkylteil und 1 bis 5 Kohlenstoffatomen im Alkoxy- und Acylteil, Hydroxyl, Halogen, Alkoxy mit 1 bis 5 Kohlenstoffatomen, Amino (—$NH_2$), N-Alkylamino und N,N-Dialkylamino mit je 1 bis 5 Kohlenstoffatomen im Alkylteil, Mercapto (—SH) und/oder Alkylmercapto mit 1 bis 5 Kohlenstoffatomen, Z ferner Cycloalkylen mit 3 bis 12 Kohlenstoffatomen,

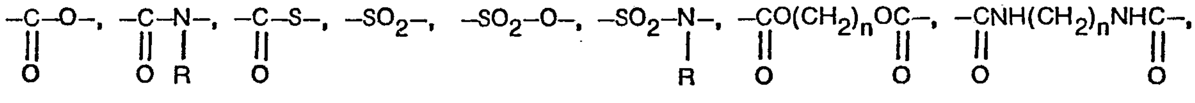

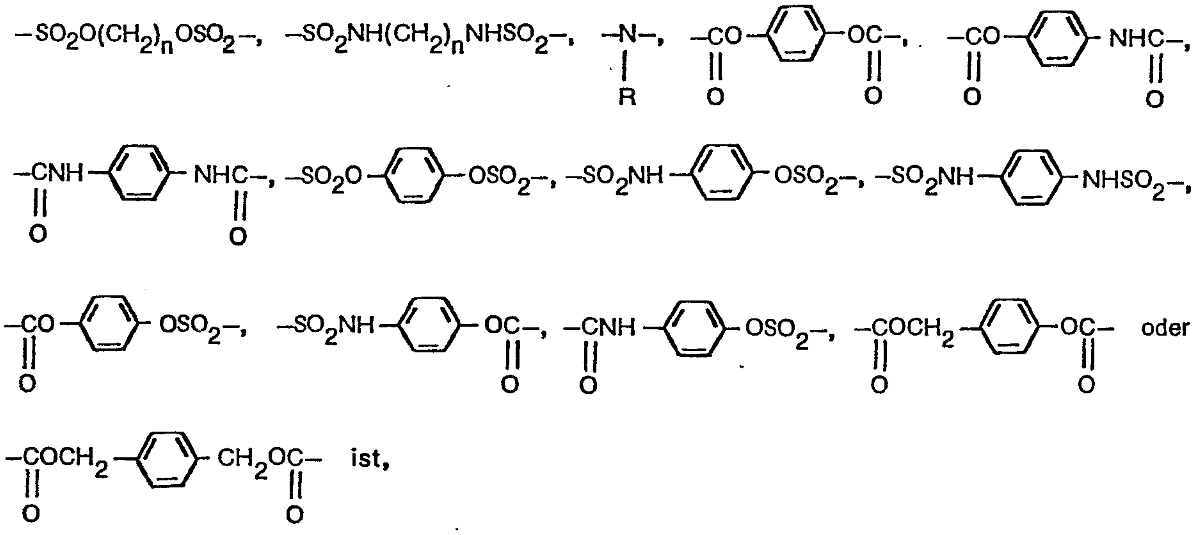

oder ist,

worin R Wasserstoff, Alkyl mit 1 bis 10 Kohlenstoffatomen, gegebenenfalls substituiert mit Halogen, Hydroxyl oder Acyl mit 2 bis 5 Kohlenstoffatomen und n eine ganze Zahl von 2 bis 20 ist, und r 1 oder 2 ist.

2. Aufzeichnungsmaterial nach Anspruch 1, dadurch gekennzeichnet, dass es mindestens einen Gelbkuppler der Formel

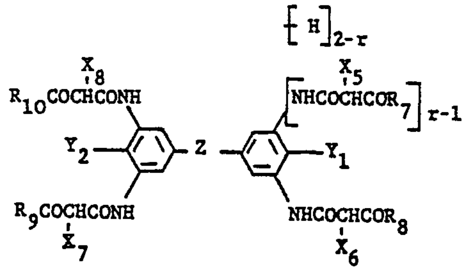

enthält, worin

$R_7$, $R_8$, $R_9$ und $R_{10}$ geradkettiges und verzweigtes Alkyl mit 1 bis 18 Kohlenstoffatomen, Cycloalkyl, Bicycloalkyl oder Tricycloalkyl mit 3 bis 12 Ringkohlenstoffatomen, Phenyl oder Phenyl substituiert mit Halogen, Alkyl oder Alkoxy mit je 1 bis 4 Kohlenstoffatomen oder substituiert mit $—O(CH_2)_rO—$, $—NH_2$, $—SO_2R_{11}$ oder $—NHCOR_{12}$, worin $R_{11}$ und $R_{12}$ je Alkyl mit 1 bis 5 Kohlenstoffatomen sind,

$X_5$, $X_6$, $X_7$ und $X_8$ Wasserstoff, Halogen, gegebenenfalls substituiertes Alkoxy oder Phenoxy, stickstoffhaltige 5- oder 6-gliedrige Heterocyclen, die über ein Stickstoffatom an die Kupplungsstelle gebunden sind, $—S—R_{13}$ worin $R_{13}$Alkyl, Phenyl oder ein Heterocyclus ist, $—OPO(OR_{14})_2$, worin $R_{14}$ Alkyl oder Phenyl ist, oder ein Rest der Formel

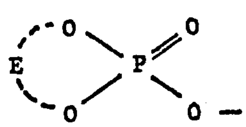

sind, worin E die Ergänzung zu einem aus dem Phosphoratom, den beiden Sauerstoffatomen und 3 Kohlenstoffatomen bestehenden sechsgliedrigen Ring ist,

$Y_1$, $Y_2$, und Z die in Anspruch 1 angegebene Bedeutung hat.

3. Aufzeichnungsmaterial nach Anspruch 2, dadurch gekennzeichnet, dass es mindestens einen Gelbkuppler der Formel

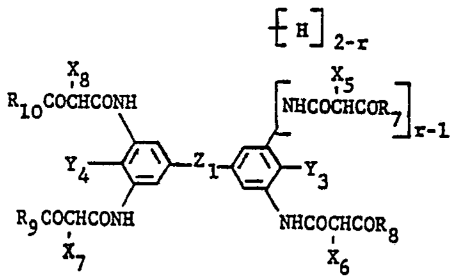

enthält worin

$Y_3$ und $Y_4$ Fluor, Chlor, Brom, Alkyl, Alkoxy und Alkylmercapto mit je 1 bis 12 Kohlenstoffatomen, —CN, —COOH, —$COOR_{15}$, —$NH_2$, —$NHR_{16}$, —$NR_{16}R_{17}$ oder —$NHCOR_{15}$ ist, worin $R_{16}$ und $R_{17}$ je Alkyl mit 1 bis 5 Kohlenstoffatomen oder Phenyl sind und $R_{15}$ Alkyl mit 1 bis 12 Kohlenstoffatomen oder Phenyl ist, $-(CH_2)_m-$, gegebenenfalls substituiert mit Alkyl und/oder Halogenalkyl mit je 1 bis 5 Kohlenstoffatomen im Alkyl- und Alkoxyteil, $Z_1$ ferner

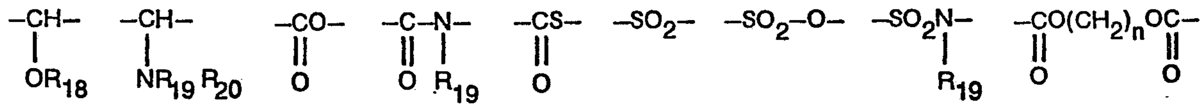

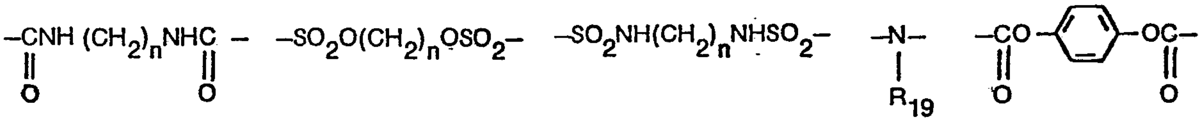

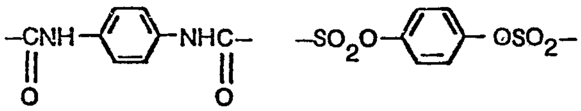 oder 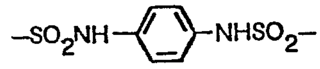 ist,

$R_{18}$ Wasserstoff oder Alkyl mit 1 bis 5 Kohlenstoffatomen ist,

$R_{19}$ und $R_{20}$ Wasserstoff oder Alkyl mit 1 bis 5 Kohlenstoffatomen gegebenenfalls substituiert mit Halogen, Hydroxyl oder Acyl mit 2 bis 5 Kohlenstoffatomen sind,

m eine ganze Zahl von 1 bis 20,

n eine ganze Zahl von 2 bis 20 ist und

$R_7$, $R_8$, $R_9$, $R_{10}$, $X_5$, $X_6$, $X_7$, $X_8$ und r die in Anspruch 2 angegebenen Bedeutungen haben.

4. Aufzeichnungsmaterial nach Anspruch 3, dadurch gekennzeichnet, dass es mindestens einen Gelbkuppler der Formel

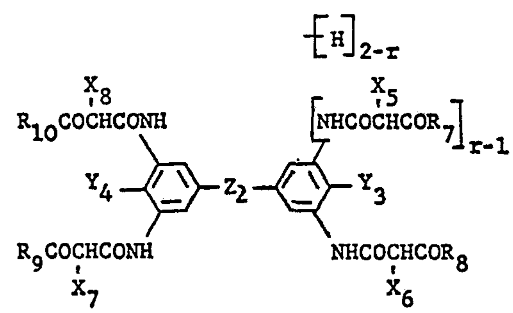

enthält, worin

$Z_2$ $-(CH_2)_m-$, 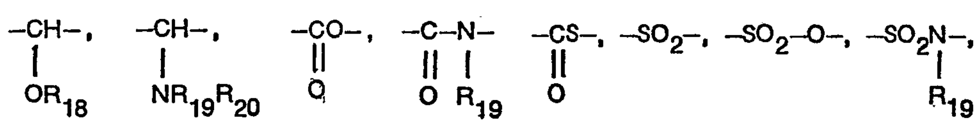

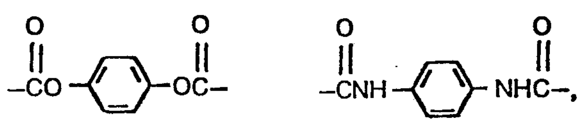 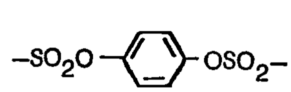 oder

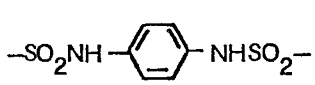 ist und

$R_7$, $R_8$, $R_9$, $R_{10}$, $X_5$, $X_6$, $X_7$, $X_8$, $Y_3$, $Y_4$, $R_{18}$, $R_{19}$, $R_{20}$, m und r die in Anspruch 3 angegebenen Bedeutungen haben.

5. Aufzeichnungsmaterial nach Anspruch 4, dadurch gekennzeichnet, dass es mindestens einen Gelbkuppler der Formel

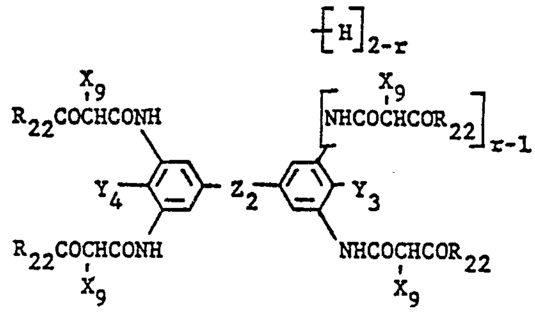

enthält, worin

$R_{22}$ geradkettiges oder verzweigtes Alkyl mit 3 bis 10 Kohlenstoffatomen, Cyclopentyl, Cyclohexyl, Adamantyl, Phenyl oder Phenyl substituiert mit Chlor oder Brom, Alkyl oder Alkoxy mit je 1 bis 4 Kohlenstoffatomen oder $-O(CH_2)_rO-$,

$X_9$ Wasserstoff, Chlor oder ein Rest der Formeln

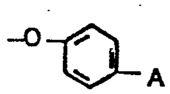 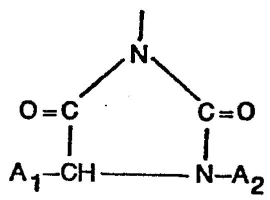 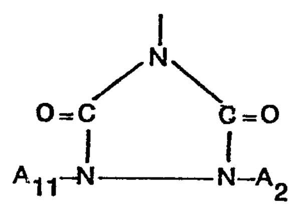 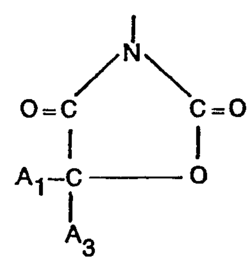

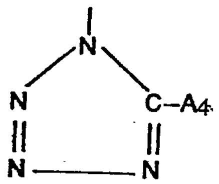 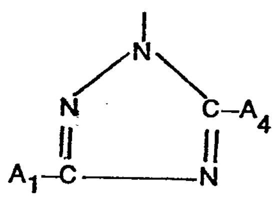 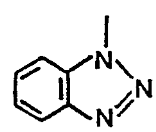 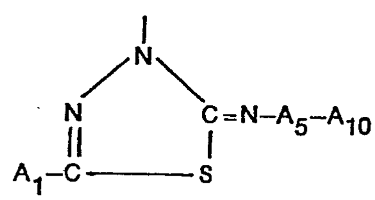

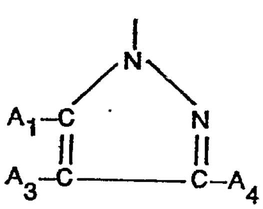 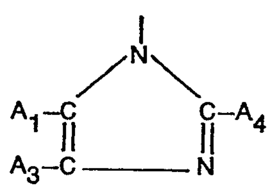 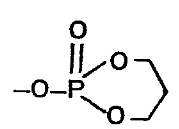 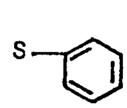 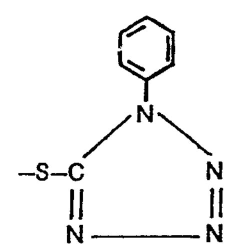

worin A COOH, $-NO_2$, $-COOR_{21}$, worin $R_{21}$ Alkyl mit 1 bis 4 Kohlenstoffatomen ist, oder der Rest der Formel

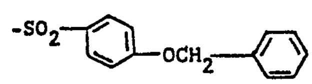 ist,

$A_1$ Wasserstoff, Alkyl mit 1 bis 18 Kohlenstoffatomen, Aralkyl, Aryl, Cycloalkyl mit einem bis vier Cycloalkylringen, Alkoxy mit 1 bis 18 Kohlenstoffatomen, Aryloxy, Alkylmercapto mit 1 bis 18 Kohlenstoffatomen, Arylmercapto, Halogen, Trifluormethyl, Cyan, $-NH_2$, Mono- oder Dialkylamino deren Alkylreste je 1 bis 18 Kohlenstoffatome enthalten,

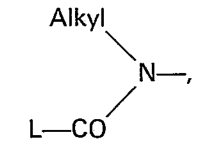

wobei Alkyl 1 bis 5 Kohlenstoffatome enthält,

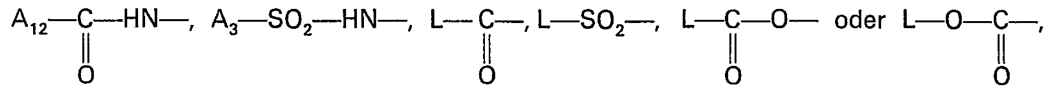

$A_2$ geradkettiges oder verzweigtes Alkyl mit 1 bis 18 Kohlenstoffatomen, Aralkyl, vorzugsweise Benzyl oder mit Alkyl, Alkoxy, Halogen, —$NH_2$, Alkylamino, Dialkylamino, Acylamino, —COOH, Carbalkoxy, Carbonamido, Sulfo, Sulfonamido oder Alkylmercapto substituiertes Phenyl,

$A_3$ unverzweigtes oder verzweigtes Alkyl mit 1 bis 18 Kohlenstoffatomen, gegebenenfalls substituiert mit Halogen, Nitro, Cyan, Alkoxy oder primären, sekundären oder tertiären Aminogruppen, Aralkyl, Cycloalkyl mit einem bis vier Cycloalkylringen; Aryl, gegebenenfalls substituiert mit Alkyl oder Alkoxy mit je 1 bis 4 Kohlenstoffatomen, Halogen, Acylamino, —$SO_3H$, —COOH, Sulfonsäureamid oder Carbonsäureamid, N- oder N,N-substituiertes Sulfonsäure- oder Carbonsäureamid, Carbonsäureester, Hydroxyl, Nitro, primärem, sekundärem oder tertiärem Amin, Mercapto, Alkylmercapto, —$SO_2$—L— oder —CO—L; Pyridyl, Furyl, Thienyl, Perfluoroalkyl, Acyl, Dialkylamino mit je 1 bis 5 Kohlenstoffatomen im Alkylteil, Alkoxy mit 1 bis 18 Kohlenstoffatomen oder Phenoxy,

$A_4$ Wasserstoff, gegebenenfalls substituiertes Alkyl mit 1 bis 18 Kohlenstoffatomen, Cycloalkyl, Cycloalkenyl, Alkenyl, Aryl, Aralkyl, ein heterocyclischer Rest, Alkoxy, Aryloxy, Alkylmercapto, gegebenenfalls durch Alkyl, Aryl oder Acyl substituiertes Amino, Alkylsulfonyl, Arylsulfonyl, Acyloxy, Aminosulfonyl, Carbonsäureamid, Sulfonsäureamid, Carbonsäurealkylester, Nitro, Cyan, Halogen, gegebenenfalls substituiertes Ureido oder gegebenenfalls substituiertes Aminosulfonylamino und

$A_5$ —CO— oder —$SO_2$— ist,

$A_{10}$ die Bedeutung von $A_3$ außer —$SO_2$L oder —COL hat und ausserdem Wasserstoff ist, sofern $A_5$ —CO— ist,

$A_{11}$ Alkyl mit 1 bis 18 Kohlenstoffatomen ist, gegebenenfalls substituiert mit Halogen, Amino, Cyano oder Alkoxy, Cycloalkyl, Aryl oder Aralkyl,

$A_{12}$ Wasserstoff ist oder die Bedeutung von $A_3$ hat,

L Alkyl mit 1 bis 18 Kohlenstoffatomen, gegebenenfalls substituiert mit Halogen, Amino, Cyano oder Alkoxy; Cycoalkyl, Aryl, Pyridyl, Pyrimidyl, Furyl oder Thienyl ist und $Y_3$, $Y_4$, $Z_2$ und r die in Anspruch 4 angegebenen Bedeutungen haben.

6. Aufzeichnungsmaterial nach Anspruch 2, dadurch gekennzeichnet, dass es mindestens einen Gelbkuppler der Formel

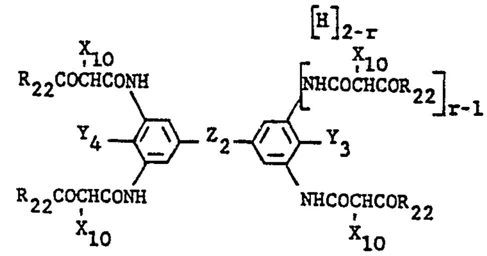

enthält, worin

$X_{10}$ Wasserstoff, Chlor oder ein Rest der Formeln

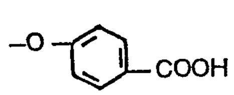 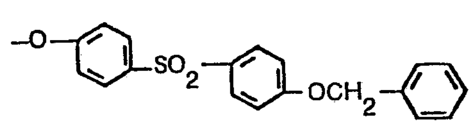 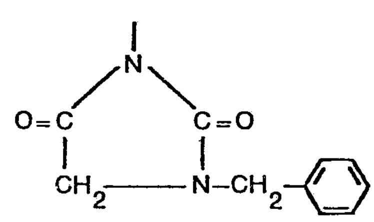

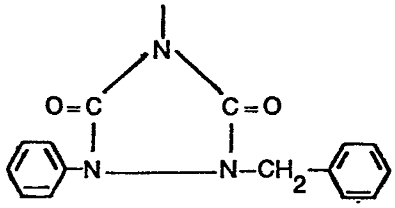  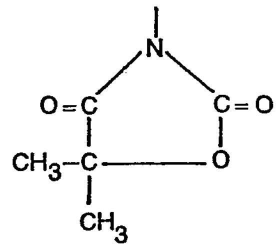  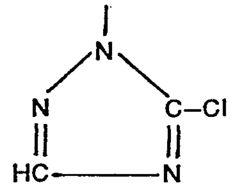

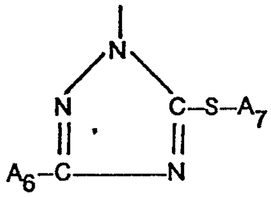  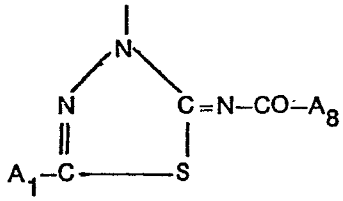  oder 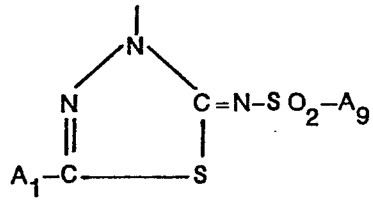 ist,

worin $A_6$ Wasserstoff oder Alkyl mit 1 bis 4 Kohlenstoffatomen, $A_7$ Alkyl mit 1 bis 12 Kohlenstoffatomen, $A_8$ geradkettiges oder verzweigtes Alkyl mit 1 bis 18 Kohlenstoffatomen,

$-CH_2Cl$  $-CCl_3$  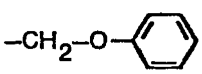  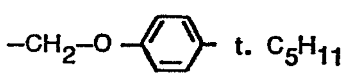  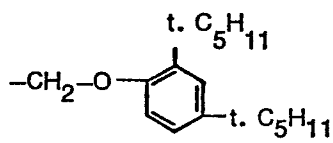

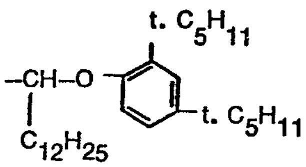  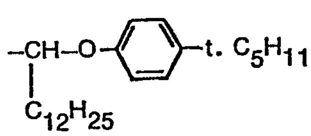  $-CH_2-O-C_pH_{2p+1}$

Aralkyl, Cycloalkyl, Phenyl oder Phenyl substituiert mit

$-C_qH_{2q+1}$  $-OC_qH_{2q+1}$  $-Cl$  $-Br$  $-NHOCC_pH_{2p+1}$  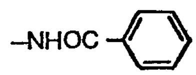  $-SO_2NH_2$

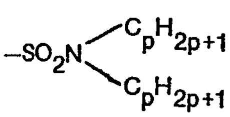  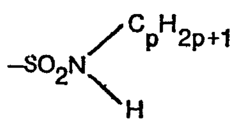  $-CONH_2$  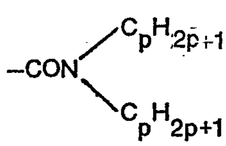  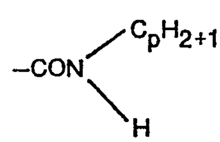

$-SO_2CH_3$  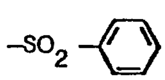  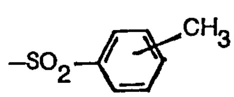  $-COOH$  $-COOC_pH_{2p+1}$  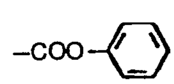

$-COO-C_pH_{2p+1}$  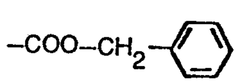  $-OH$  $-NH_2$  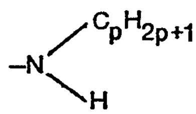  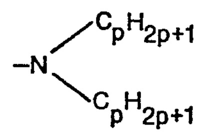

$-SC_pH_{2p+1}$  $-SO_3H$  $-COC_pH_{2p+1}$  oder  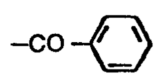

Pyridyl, Furyl, Thienyl, $-C_pF_{2p+1}$  $-COC_pH_{2p+1}$  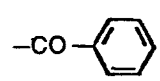  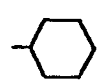  Norbornyl,

Adamantyl, 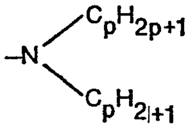  $-O-C_pH_{2p+1}$  oder  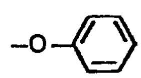

P eine Zahl von 1 bis 5, q eine Zahl von 1 bis 4 und $A_9$ $-CH_3$ 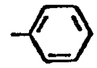 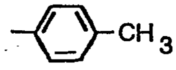

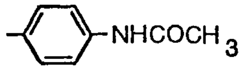 und

$R_{22}$ geradkettiges oder verzweigtes Alkyl mit 3 bis 10 Kohlenstoffatomen, Cyclopentyl, Cyclohexyl, Adamantyl, Phenyl oder Phenyl substituiert mit Chlor oder Brom, Alkyl oder Alkoxy mit je 1 bis 4 Kohlenstoffatomen oder $-O(CH_2)_rO-$, und $Y_3$, $Y_4$, $Z_2$ und r die in Anspruch 2 angegebene Bedeutung haben.

7. Farbphotographisches Verfahren zur Herstellung eines Gelbbildes durch Farbentwicklung eines bildmässig belichteten Aufzeichnungsmaterial nach einem der Ansprüche 1 bis 6.

8. Verbindungen der Formel

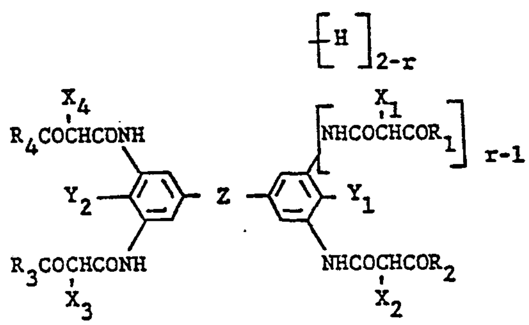

worin

$R_1$, $R_2$, $R_3$ und $R_4$ Alkyl, Cycloalkyl oder Aryl,

$X_1$, $X_2$, $X_3$ und $X_4$ während einer Kupplungsreaktion abspaltbare Reste, und

$Y_1$ und $Y_2$ Halogen, Alkyl, Alkoxy, Alkylmercapto, —CN, —COOH, Carbalkoxy, $-NH_2$, $-NHR_5$, $-NR_5R_6$ oder $-NHCOR_5$ sind, worin $R_5$ und $R_6$ Alkyl oder Phenyl sind,

Z ein geradkettiges Alkylen mit 1 bis 20 Kohlenstoffatomen, gegebenenfalls substituiert mit Alkyl, Hydroxyalkyl, Alkoxyalkyl, Aminoalkyl, Acylaminoacyl oder Halogenalkyl mit je 1 bis 10 Kohlenstoffatomen mit Alkylteil und 1 bis 5 Kohlenstoffatomen im Alkoxy- und Acylteil, Hydroxyl, Halogen, Alkoxy mit 1 bis 5 Kohlenstoffatomen, Amino ($-NH_2$), N-Alkylamino und N,N-Dialkylamino mit je 1 bis 5 Kohlenstoffatomen im Alkylteil, Mercapto (—SH) und/oder Alkylmercapto mit 1 bis 5 Kohlenstoffatomen, Z ferner Cycloalkylen mit 3 bis 12 Kohlenstoffatomen,

$-\underset{\underset{O}{\|}}{C}-O-$, $-\underset{\underset{O}{\|}}{C}-\underset{\underset{R}{|}}{N}-$, $-\underset{\underset{O}{\|}}{C}-S-$, $-SO_2-$, $-SO_2-O-$, $-SO_2-\underset{\underset{R}{|}}{N}-$, $-\underset{\underset{O}{\|}}{C}O(CH_2)_nO\underset{\underset{O}{\|}}{C}-$, $-\underset{\underset{O}{\|}}{C}NH(CH_2)_nNH\underset{\underset{O}{\|}}{C}-$,

$-SO_2O(CH_2)_nOSO_2-$, $-SO_2NH(CH_2)_nNHSO_2-$, $-\underset{\underset{R}{|}}{N}-$, 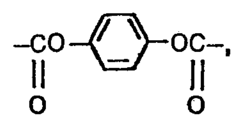, 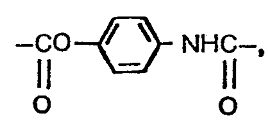,

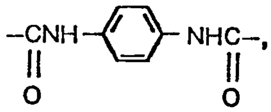, 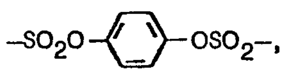, 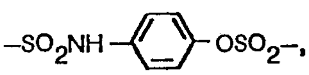, 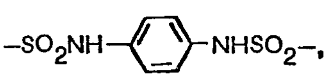,

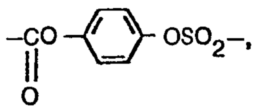, 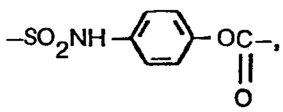, 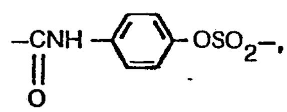,

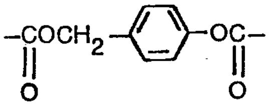 oder 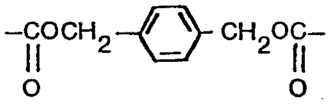 ist,

worin R Wasserstoff, Alkyl mit 1 bis 10 Kohlenstoffatomen, gegebenenfalls substituiert mit Halogen, Hydroxyl oder Acyl mit 2 bis 5 Kohlenstoffatomen und n eine ganze Zahl von 2 bis 20 ist, und r 1 oder 2 ist.

9. Verfahren zur Herstellung von Verbindungen gemäss Anspruch 8, dadurch gekennzeichnet, dass man Tri- oder Tetranitroverbindungen der Formel

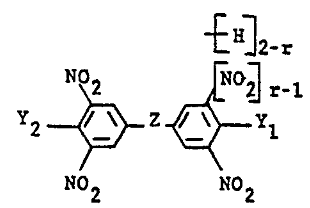

worin $Y_1$, $Y_2$, Z und r die in Anspruch 8 angegebenen Bedeutungen haben, zu den entsprechenden Aminoverbindungen reduziert, mit 3 oder 4 Mol von Estern der Formeln $RCOCH_2COOAlk$, worin R die Bedeutung von $R_1$ bis $R_4$ in Anspruch 8 hat, umsetzt, gegebenenfalls ein Wasserstoffatom in den $—CH_2—$ Gruppen durch ein Halogenatom ersetzt und die so erhaltenen Verbindungen mit Salzen der Formel $M^{\oplus}X^{\ominus}$, worin $M^{\oplus}$ ein Kation ist und X die Bedeutung von $X_1$ bis $X_4$ in Anspruch 8 hat, umsetzt.

10. Verwendung von Verbindungen gemäss Anspruch 8 als Gelbkuppler in farbphotographischen Auszeichnungsmaterialien.

## Claims

1. A recording material for colour photography, which material contains, in at least one silver halide emulsion layer, at least one yellow coupler having several groups detachable during a coupling reaction, characterised in that the yellow coupler of the formula

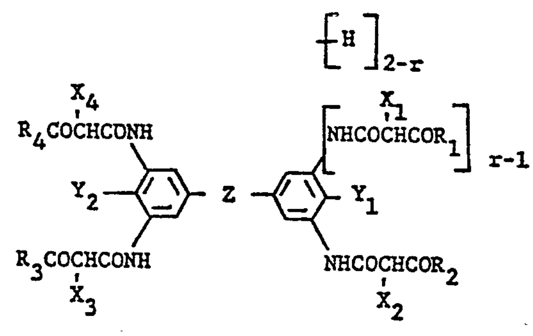

wherein

$R_1$, $R_2$, $R_3$ and $R_4$ are alkyl, cycloalkyl or aryl,

$X_1$, $X_2$, $X_3$ and $X_4$ are radicals detachable during a coupling reaction, and

$Y_1$ and $Y_2$ are halogen, alkyl, alkoxy, alkylmercapto, —CN, —COOH, carbalkoxy, $—NH_2$, $—NHR_5$, $—NR_5R_6$ or $—NHCOR_5$, in which $R_5$ and $R_6$ are alkyl or phenyl,

Z is a straight-chain alkylene group having 1 to 20 carbon atoms, which is unsubstituted or substituted by alkyl, hydroxyalkyl, alkoxyalkyl, aminoalkyl, acylaminoalkyl or haloalkyl having 1 to 10 carbon atoms in each alkyl moiety, and 1 to 5 carbon atoms in each alkoxy and acyl moiety, or Z is hydroxyl, halogen, alkoxy having 1 to 5 carbon atoms, amino ($—NH_2$), N-alkylamino and N,N-dialkylamino having 1 to 5 carbon atoms in each alkyl moiety, mercapto (—SH) and/or alkylmercapto having 1 to 5 carbon atoms, cycloalkylene having 3 to 12 carbon atoms,

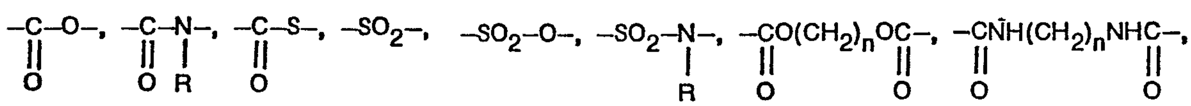

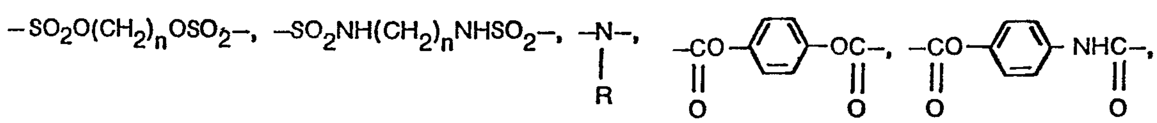

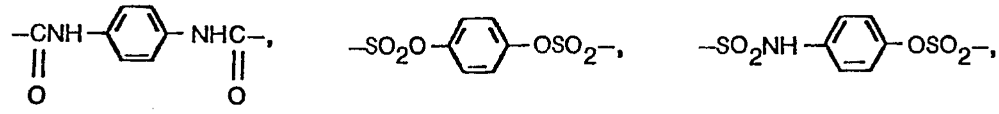

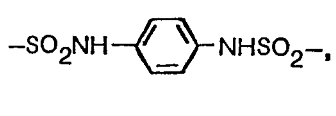, 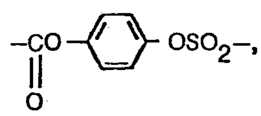, 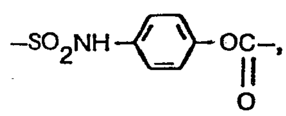,

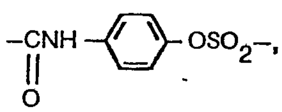, 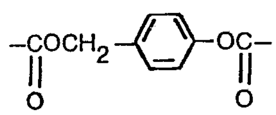 or 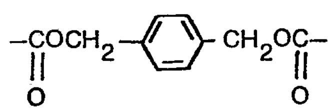

wherein R is hydrogen, alkyl having 1 to 10 carbon atoms, which is unsubstituted or substituted by halogen, hydroxyl or acyl having 2 to 5 carbon atoms, and n is an integer from 2 to 20, and

r is 1 or 2.

2. A recording material according to Claim 1, characterised in that it contains at least one yellow coupler of the formula

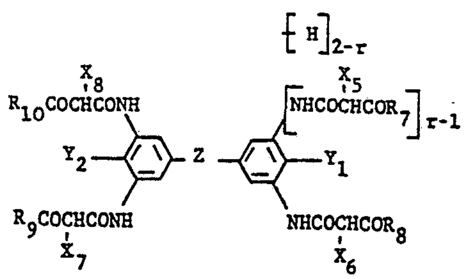

wherein

$R_7$, $R_8$, $R_9$ and $R_{10}$ are straight-chain and branched-chain alkyl having 1 to 18 carbon atoms, cycloalkyl, bicycloalkyl or tricycloalkyl having 3 to 18 ring carbon atoms, phenyl, or phenyl substituted by halogen, alkyl or alkoxy each having 1 to 4 carbon atoms, or substituted by $—O(CH_2)_rO—$, $—NH_2$, $—SO_2R_{11}$ or $—NHCOR_{12}$, in which $R_{11}$ and $R_{12}$ are each alkyl having 1 to 5 carbon atoms,

$X_5$, $X_6$, $X_7$ and $X_8$ are hydrogen, halogen, unsubstituted or substituted alkoxy or phenoxy, nitrogen-containing 5- or 6-membered heterocycles which are bound by way of a nitrogen atom to the coupling position, $—S—R_{13}$, in which $R_{13}$ is alkyl, phenyl or a heterocycle, $—OPO(OR_{14})_2$, in which $R_{14}$ is alkyl or phenyl, or a radical of the formula

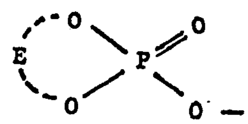

wherein E is the remaining part of a six-membered ring consisting of the phosphorus atom, the two oxygen atoms and 3 carbon atoms, and

$Y_1$, $Y_2$, r and Z have the meanings given in Claim 1.

3. A recording material according to Claim 2, characterised in that it contains at least one yellow coupler of the formula

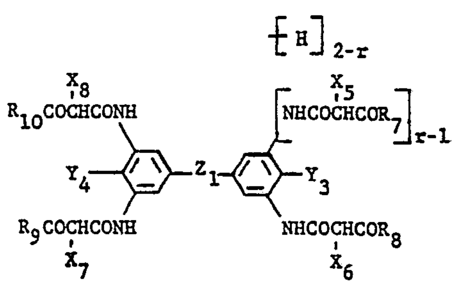

wherein

$Y_3$ and $Y_4$ are fluorine, chlorine, bromine, alkyl, alkoxy and alkylmercapto, each having 1 to 12 carbon atoms, $—CN$, $—COOH$, $—COOR_{15}$, $—NH_2$, $—NHR_{16}$, $—NR_{16}R_{17}$ or $—NHCOR_{15}$, in which $R_{16}$ and $R_{17}$ are each alkyl having 1 to 5 carbon atoms or are phenyl, and $R_{15}$ is alkyl having 1 to 12 carbon atoms or is phenyl,

$Z_1$ is $-(CH_2)_m-$, which is unsubstituted or substituted by alkyl, hydroxyalkyl, alkoxyalkyl, aminoalkyl and/or haloalkyl, having 1 to 5 carbon atoms in each alkyl and alkoxy moiety, and $Z_1$ is also

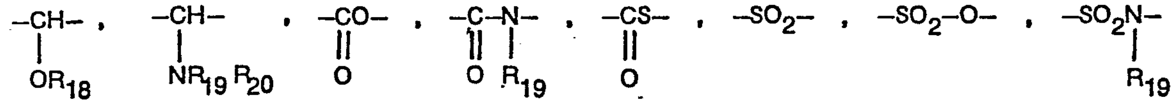

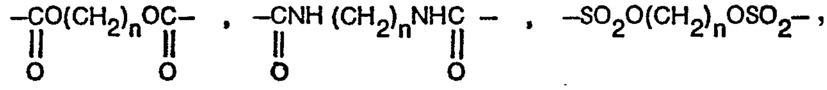

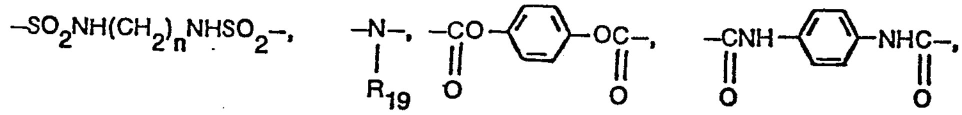

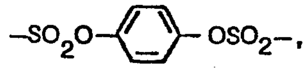 or 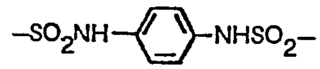

in which $R_{18}$ is hydrogen or alkyl having 1 to 5 carbon atoms, $R_{19}$ and $R_{20}$ are hydrogen or alkyl having 1 to 5 carbon atoms, which is unsubstituted or substituted by halogen, hydroxyl or acyl having 2 to 5 carbon atoms, m is an integer from 1 to 20, and n is an integer from 2 to 20, and

$R_7$, $R_8$, $R_9$, $R_{10}$, $X_5$, $X_6$, $X_7$, $X_8$ and r have the meanings defined in Claim 2.

4. A recording material according to Claim 3, characterised in that it contains at least one yellow coupler of the formula

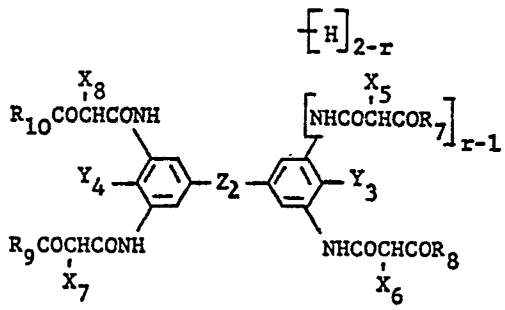

wherein

$Z_2$ is 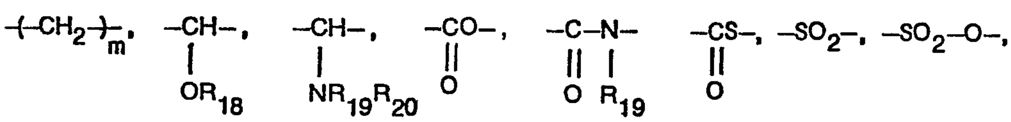

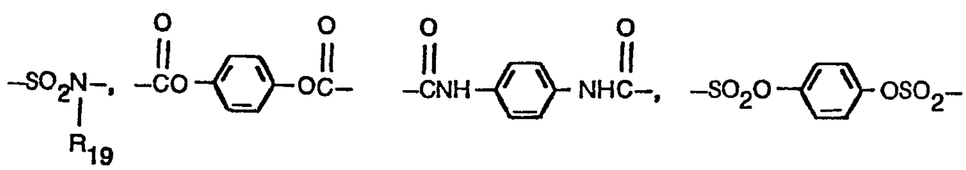 or

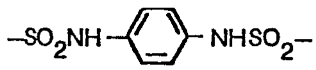 and

$R_7$, $R_8$, $R_9$, $R_{10}$, $X_5$, $X_6$, $X_7$, $X_8$, $Y_3$, $Y_4$, $R_{18}$, $R_{19}$, $R_{20}$, m and r have the meanings given in Claim 3.

5. A recording material according to Claim 4, characterised in that it contains at least one yellow coupler of the formula

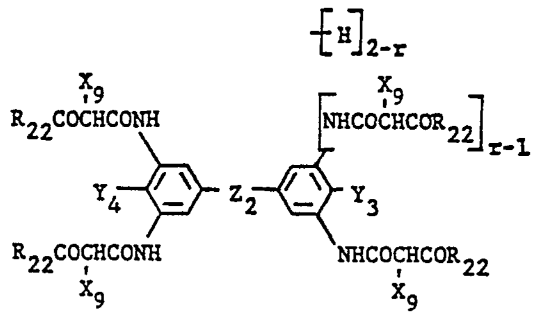

wherein

$R_{22}$ is straight-chain or branched-chain alkyl having 3 to 10 carbon atoms, cyclopentyl, cyclohexyl, adamantyl, phenyl, or phenyl substituted by chlorine or bromine, or it is alkyl or alkoxy, each having 1 to 4 carbon atoms, or —$O(CH2)_4O$—,

$X_9$ is hydrogen, chlorine or a radical of the formulae

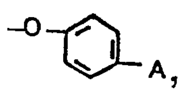, 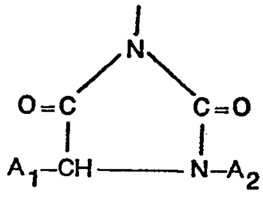, 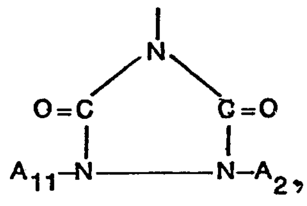, 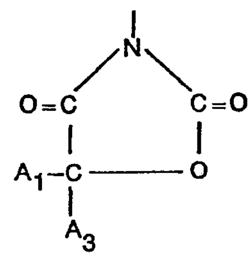,

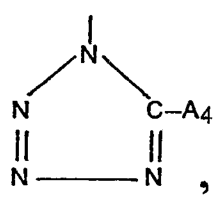, 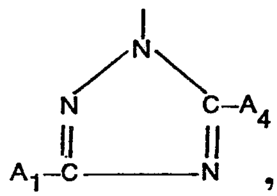, 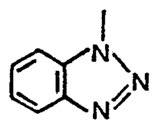, 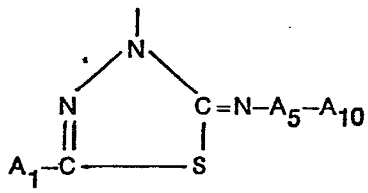,

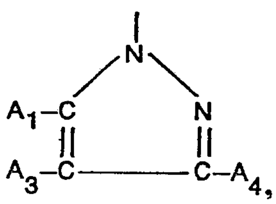, 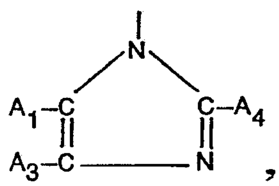, 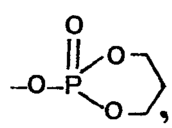, 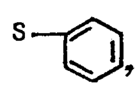, 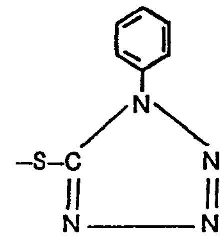,

in which

A is —COOH, —$NO_2$ or —$COOR_{21}$, wherein $R_{21}$ is alkyl having 1 to 4 carbon atoms, or a radical of the formula

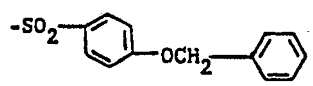

$A_1$ is hydrogen, alkyl having 1 to 18 carbon atoms, aralkyl, aryl, cycloalkyl having one to four cycloalkyl rings, alkoxy having 1 to 18 carbon atoms, aryloxy, alkylmercapto having 1 to 18 carbon atoms, arylmercapto, halogen trifluoromethyl, cyano, —$NH_2$ or mono- or dialkylamino, of which the alkyl radicals each contain 1 to 18 carbon atoms,

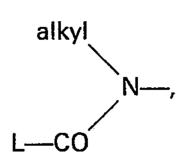

in which alkyl contains 1 to 5 carbon atoms,

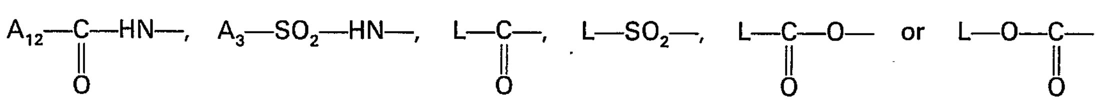

or

$A_2$ is straight-chain or branched-chain alkyl having 1 to 18 carbon atoms, aralkyl, preferably benzyl, or phenyl substituted by alkyl, alkoxy, halogen, —$NH_2$ alkylamino, dialkylamino, acylamino, —COOH, carbalkoxy, carbonamido, sulfo, sulfonamido or alkylmercapto,

$A_3$ is straight-chain or branched-chain alkyl having 1 to 18 carbon atoms, which is unsubstituted or substituted by halogen, nitro, cyano, alkoxy or primary, secondary or tertiary amino groups, aralkyl or cycloalkyl having one to four cycloalkyl rings; aryl, which is unsubstituted or substituted by alkyl or alkoxy each having 1 to 4 carbon atoms, halogen, acylamino, —$SO_3H$, —COOH, sulfonamide or carboxamide, N— or N,N-substituted sulfonamide or carboxamide, carboxylic acid ester, hydroxyl, nitro, primary, secondary or tertiary amine, mercapto, alkylmercapto, —$SO_2$—L or —CO—L; pyridyl, furyl,

thienyl, perfluoroalkyl, acyl or dialkylamino, each having 1 to 5 carbon atoms in the alkyl moiety, alkoxy having 1 to 18 carbon atoms, or phenoxy,

$A_4$ is hydrogen, unsubstituted or substituted alkyl having 1 to 18 carbon atoms, cycloalkyl, cycloalkenyl, alkenyl, aryl, aralkyl, a heterocyclic radical, alkoxy, aryloxy, alkylmercapto, amino which is unsubstituted or substituted by alkyl, aryl or acyl, or it is alkylsulfonyl, arylsulfonyl, acyloxy, aminosulfonyl, carboxamide, sulfonamide, alkyl carboxylate, nitro, cyano, halogen, unsubstituted or substituted aminosulfonylamino, and

$A_5$ is —CO— or —$SO_2$—,

$A_{10}$ has the meaning defined for $A_3$ except —$SO_2L$ and —CO—L, and is also hydrogen if $A_5$ is —CO—,

$A_{11}$ is alkyl having 1 to 18 carbon atoms, which is unsubstituted or substituted by halogen, amino, cyano or alkoxy, or it is cycloalkyl, aryl or aralkyl,

$A_{12}$ is hydrogen or has the meaning of $A_3$, and

L is alkyl having 1 to 18 carbon atoms, which is unsubstituted or substituted by halogen, amino, cyano or alkoxy; or it is cycloalkyl, aryl, pyridyl, pyrimidyl, furyl or thienyl, and

$Y_3$, $Y_4$, $Z_2$ and r have the meanings defined in Claim 4.

6. A recording material according to Claim 2, characterised in that it contains at least one yellow coupler of the formula

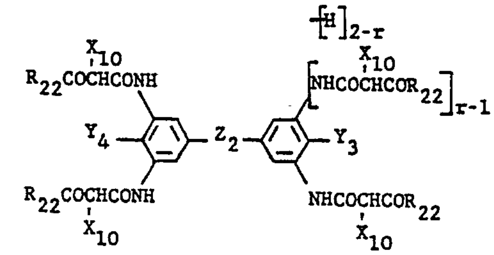

wherein

$X_{10}$ is hydrogen, chlorine or a radical of the formulae

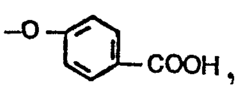, 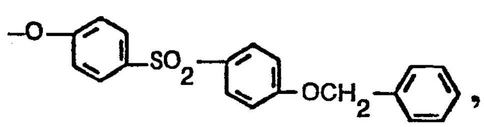, 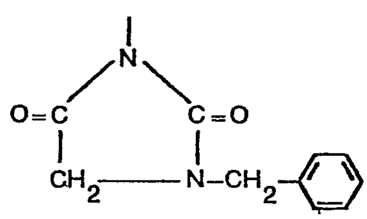,

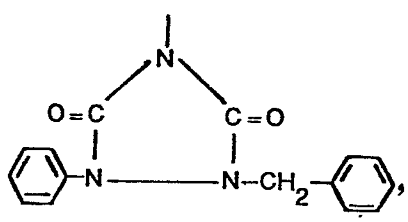, 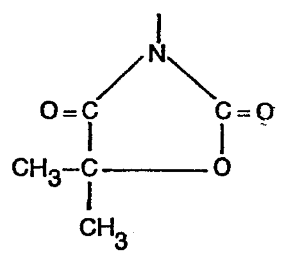, 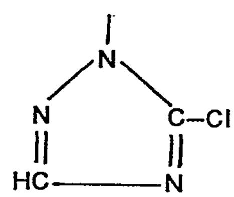,

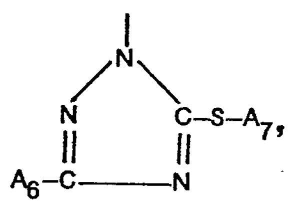, 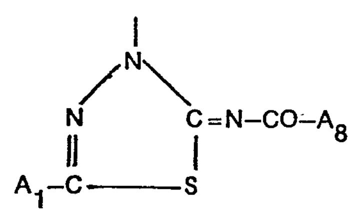 or 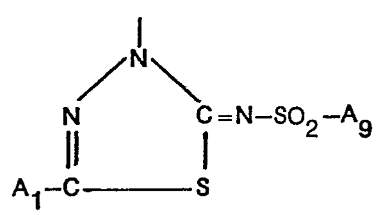

wherein

$A_6$ is hydrogen or alkyl having 1 to 4 carbon atoms,

$A_7$ is alkyl having 1 to 12 carbon atoms,

$A_8$ is stright-chain or branched-chain alkyl having 1 to 18 carbon atoms,

$-CH_2Cl$, $-CCl_3$, 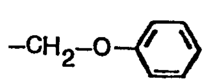, 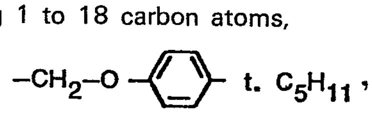,

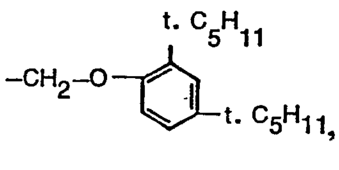 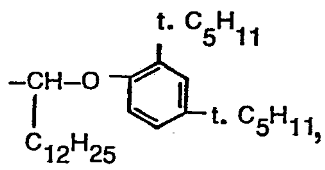 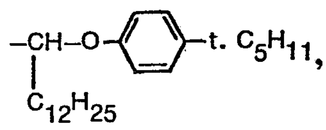

$-CH_2-O-C_pH_{2p+1}$,

aralkyl, cycloalkyl, phenyl or phenyl substituted by

$-C_qH_{2q+1}$, $-OC_qH_{2q+1}$, $-Cl$, $-Br$, $-NHOCC_pH_{2p+1}$,

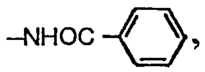, $-SO_2NH_2$, 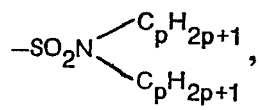, 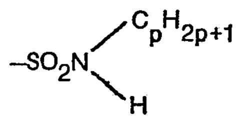, $-CONH_2$,

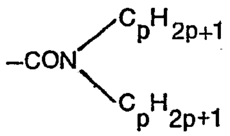, 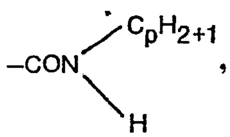, $-SO_2CH_3$, 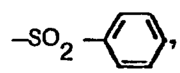, 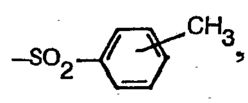,

$-COOH$, $-COOC_pH_{2p+1}$, 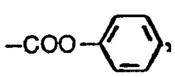, $-COO-C_pH_{2p+1}$, 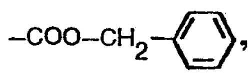, $-OH$,

$-NH_2$, 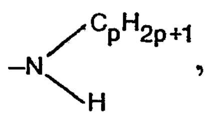, 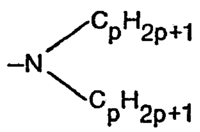, $-SC_pH_{2p+1}$, $-SO_3H$,

$-COC_pH_{2p+1}$ or 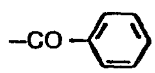

Pyridyl, Furyl, Thienyl, $-C_pF_{2p+1}$, $-COC_pH_{2p+1}$,

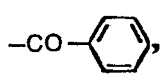, 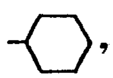, Norbornyl, Adamantyl,

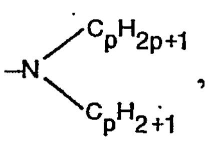, $-O-C_pH_{2p+1}$ or 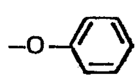

p is a number from 1 to 5,

q is a number from 1 to 4, and

$A_9$ is

$-CH_3$, 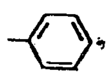, 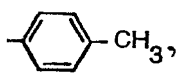, 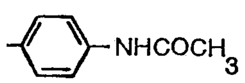 and

$R_{22}$ is straight-chain or branched-chain alkyl having 3 to 10 carbon atoms, cyclopentyl, cyclohexyl, adamantyl, phenyl, or phenyl substituted by chlorine or bromine, or it is alkyl or alkoxy each having 1 to 4 carbon atoms, or $-O(CH_2)_rO-$, and

$Y_3$, $Y_4$, $Z_2$ and r have the meanings defined in Claim 2.

7. A process, for colour photography, for the production of a yellow image by colour development of a recording material according to any one of Claims 1 to 6, which material has been exposed imagewise.

8. A compound of the formula

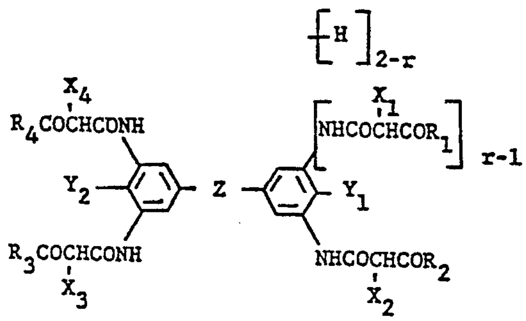

wherein

$R_1$, $R_2$, $R_3$ and $R_4$ are alkyl, cycloalkyl or aryl,

$X_1$, $X_2$, $X_3$ and $X_4$ are radicals detachable during the coupling reaction, and

$Y_1$ and $Y_2$ are halogen, alkyl, alkoxy, alkylmercapto, —CN, —COOH, carbalkoxy, —$NH_2$, —$NHR_5$, —$NR_5R_6$ or $NHCOR_5$, in which $R_5$ and $R_6$ are alkyl or phenyl,

Z is a straight-chain alkylene group having 1 to 20 carbon atoms, which is unsubstituted or substituted by alkyl, hydroxyalkyl, alkoxyalkyl, aminoalkyl, acylaminoacyl or haloalkyl having 1 to 10 carbon atoms in each alkyl moiety, and 1 to 5 carbon atoms in each alkoxy and acyl moiety, or Z is hydroxyl, halogen, alkoxy having 1 to 5 carbon atoms, amino (—$NH_2$), N-alkylamino and N,N-dialkylamino having 1 to 5 carbon atoms in each alkyl moiety, mercapto (—SH) and/or alkylmercapto having 1 to 5 carbon atoms, cycloalkylene having 3 to 12 carbon atoms,

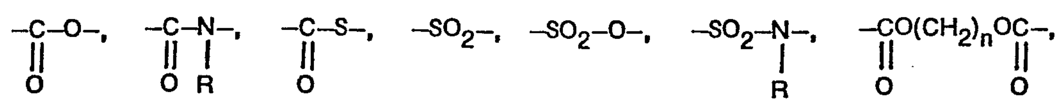

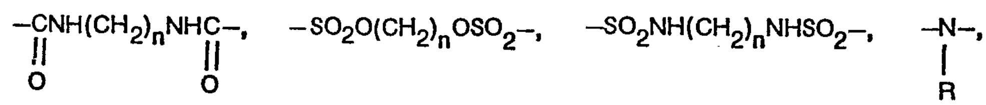

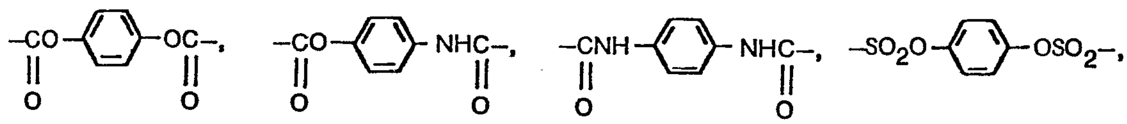

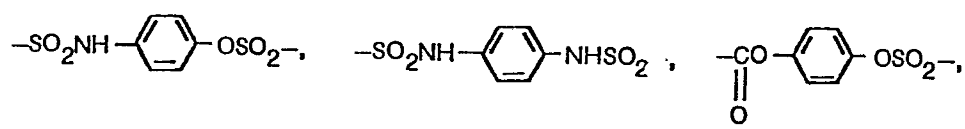

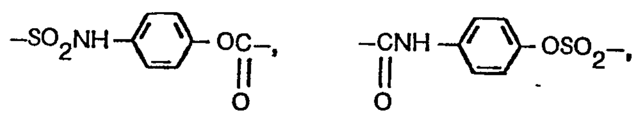

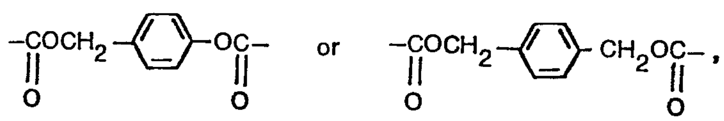

wherein R is hydrogen, alkyl having 1 to 10 carbon atoms, which is unsubstituted or substituted by halogen, hydroxyl or acyl having 2 to 5 carbon atoms, and n is an integer from 2 to 20, and

r is 1 or 2.

9. A process for producing a compound according to Claim 8, which process comprises reducing a tri- or tetranitro compound of the formula

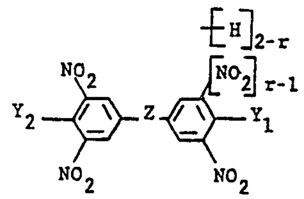

wherein $Y_1$, $Y_2$, Z and r have the meanings defined in Claim 8, to the corresponding amino compound, reacting this with 3 or 4 mols of an ester of the formula $RCOCH_2COO$ alkyl, in which R has the meaning of $R_1$ to $R_4$ in Claim 8, optionally replacing a hydrogen atom in the $-CH_2$ group by a halogen atom, and reacting the resulting compound with a salt of the formula $M^{\oplus}X^{\ominus}$, wherein $M^{\oplus}$ is a cation, and X has the meaning of $X_1$ to $X_4$ in Claim 8.

10. Use of a compound according to Claim 8 as a yellow coupler in recording material for colour photography.

**Revendications**

1. Matière d'enregistrement photographique en couleur, renfermant, dans au moins une couche d'émulsion d'halogénure d'argent, au moins un coupleur jaune avec plusieurs groupes pouvant être éliminés lors d'une réaction de copulation, caractérisée par le fait que le coupleur jaune répond à la formule

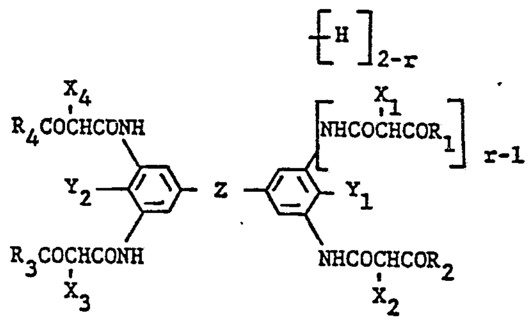

où

$R_1$, $R_2$, $R_3$ et $R_4$ désignent alkyle, cycloalkyle ou aryle,

$X_1$, $X_2$, $X_3$ et $X_4$ désignent des restes pouvant être éliminés au cours d'une réaction de copulation, et

$Y_1$ et $Y_2$ désignent halogène, alkyle, alcoxy, alkylmercapto, $-CN$, $-COOH$, carbalcoxy, $-NH_2$, $-NHR_5$, $-NR_5R_6$ ou $-NHCOR_5$, où $R_5$ et $R_6$ désignent alkyle ou phényle, et un alkylène à chaîne droite ayant de 1 à 20 atomes de carbone, éventuellement substitué par alkyle, hydroxyalkyle, alcoxyalkyle, aminoalkyle, acylaminoalkyle ou halogénoalkyle, ayant chacun de 1 à 10 atomes de carbone dans la partie alkyle et 1 à 5 atomes de carbone dans la partie alcoxy et acyle, hydroxyle, halogène, alcoxy ayant de 1 à 5 atomes de carbone, amino ($-NH_2$), N-alkylamino et N,N-dialkylamino ayant chacun de 1 à 5 atomes de carbone dans la partie alkyle, mercapto ($-SH$) et/ou alkylmercapto ayant de 1 à 5 atomes de carbone, Z peut en outre désigner cycloalkylène ayant de 3 à 12 atomes de carbone.

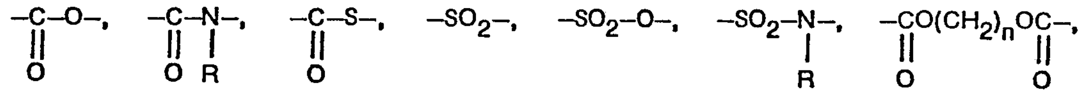

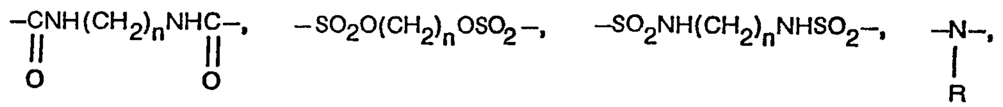

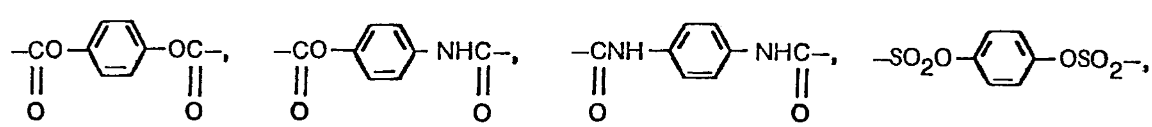

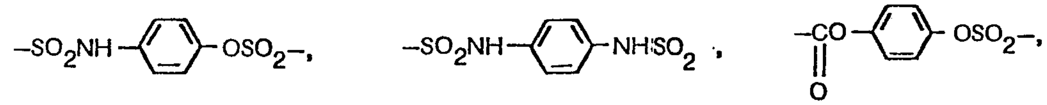

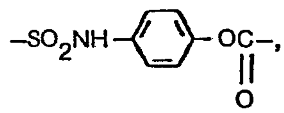
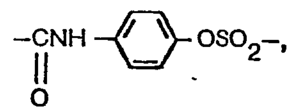

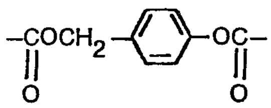
ou
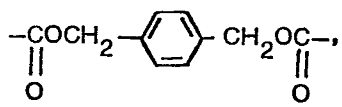

où R désigne hydrogène, alkyle ayant de 1 à 10 atomes de carbone, éventuellement substitué par halogène, hydroxyle ou acyle ayant de 2 à 5 atomes de carbone et n est un nombre entier de 2 à 20, r est 1 ou 2.

2. Matière d'enregistrement selon la revendication 1, caractérisée par le fait qu'elle renferme au moins un coupleur jaune de formule

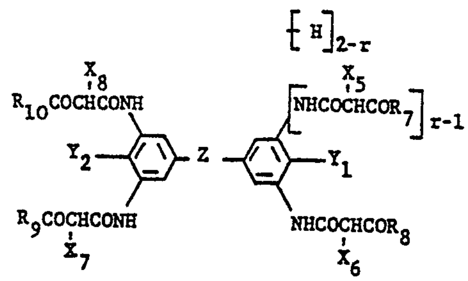

où

$R_7$, $R_8$, $R_9$ et $R_{10}$ désignent alkyle à chaîne droite ou ramifiée ayant de 1 à 18 atomes de carbone, cycloalkyle, bicycloalkyle ou tricycloalkyle ayant de 3 à 12 atomes de carbone dans le noyeaux, phényle ou phényle substitué par halogène, alkyle ou alcoxy ayant chacun de 1 à 4 atomes de carbone ou bien substitué par $—O(CH_2)_rO—$, $—NH_2$, $—SO_2R_{11}$ ou $—NHCOR_{12}$, où $R_{11}$ et $R_{12}$ désignent chacun alkyle ayant de 1 à 5 atomes de carbone,

$X_5$, $X_6$, $X_7$ et $X_8$ désignent hydrogène, halogène, alcoxy ou phénoxy éventuellement substitués, hétérocycles contenant de l'azote, à 5 ou 6 chaînons, liés à l'emplacement de copulation par l'intermédiaire d'un atome d'azote, $—S—R_{13}$ où $R_{13}$ désigne alkyle, phényle ou un hétérocycle, $—OPO(OR_{14})_2$, où $R_{14}$ désigne alkyle ou phényle, ou un reste de formule,

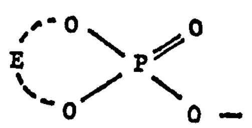

où E représente le complément à un noyau à 6 chaînons, constitué de l'atome de phosphore, des deux atomes d'oxygène et de 3 atomes de carbone,

$Y_1$, $Y_2$ et Z ont les significations indiquées dans la revendication 1.

3. Matière selon la revendication 2, caractérisée par le fait qu'elle contient au moins un coupleur jaune de formule

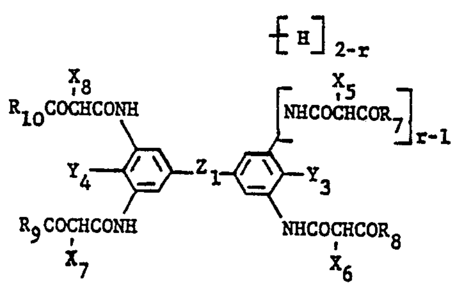

où

$Y_3$ et $Y_4$ désignent fluor, chlore, brome, alkyle, alcoxy et alkylmercapto ayant chacun de 1 à 12 atomes de carbone, $—CN$, $—COOH$, $—COOR_{15}$, $—NH_2$, $—NHR_{16}$, $—NR_{16}R_{17}$ ou $—NHCOR_{15}$, où $R_{16}$ et $R_{17}$ désignent chacun alkyle ayant de 1 à 5 atomes de carbone ou phényle et $R_{15}$ désigne alkyle ayant de 1 à 12 atomes de carbone ou phényle,

$Z_1$ désigne $-(CH_2)_m-$, éventuellement substitué par alkyle, hydroxyalkyle, alcoxyalkyle, aminoalkyle et/ou halogénoalkyle, ayant chacun de 1 à 5 atomes de carbone dans la partie alkyle et alcoxy, $Z_1$ désigne en outre

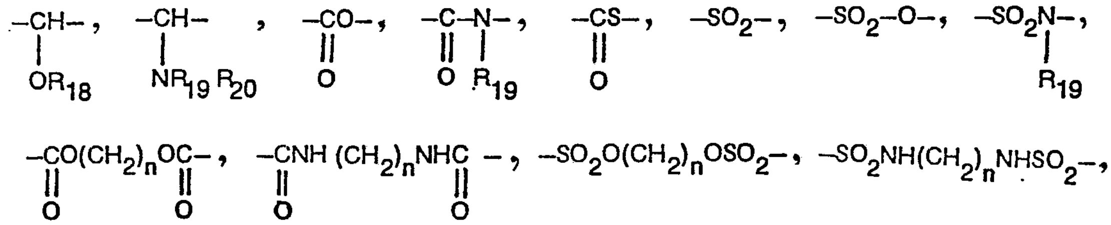

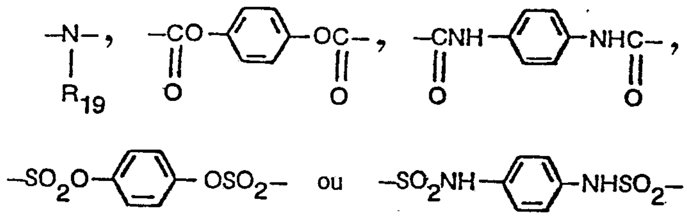

ou

$R_{18}$ désigne hydrogène ou alkyle ayant de 1 à 5 atomes de carbone,

$R_{19}$ et $R_{20}$ désignent hydrogène ou alkyle ayant de 1 à 5 atomes de carbone, éventuellement substitué par halogène, hydroxyle ou acyle ayant de 2 à 5 atomes de carbone,

m désigne un nombre entier de 1 à 20,

n désigne un nombre entier de 2 à 20, et

$R_8$, $R_8$, $R_9$, $R_{10}$, $X_5$, $X_6$, $X_7$, $X_8$ et r ont les significations indiquées dans la revendication 2.

4. Matière d'enregistrement selon la revendication 3, caractérisée par le fait qu'elle renferme au moins un coupleur jaune de formule

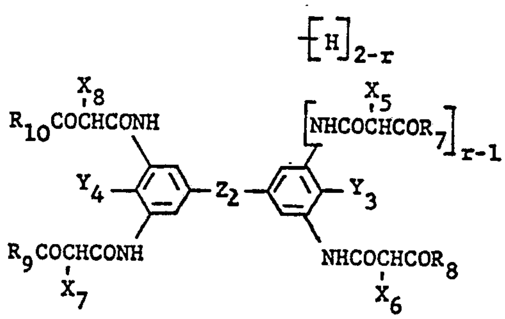

où

$Z_2$ désigne

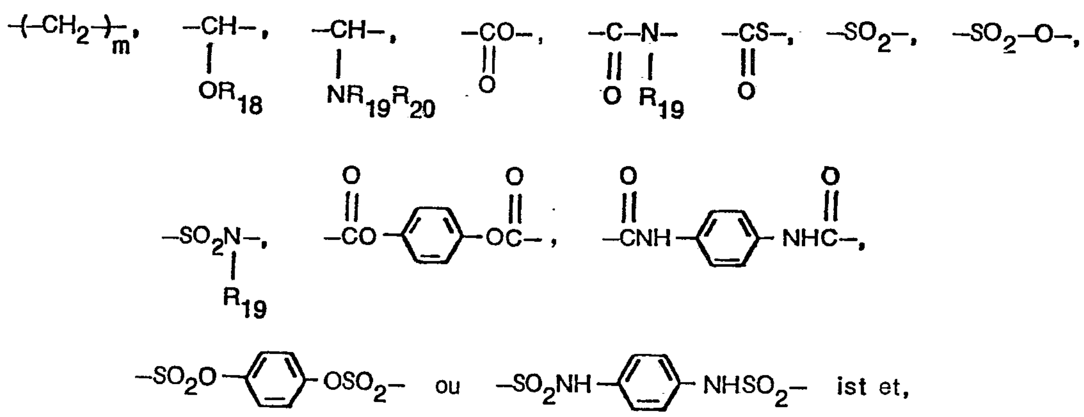

ou ist et,

$R_7$, $R_8$, $R_9$, $R_{10}$, $X_5$, $X_6$, $X_7$, $X_8$, $Y_3$, $Y_4$, $R_{18}$, $R_{19}$, $R_{20}$, m et r ont les significations indiquées dans la revendication 3.

5. Matière d'enregistrement selon la revendication 4, caractérisée par le fait qu'elle renferme au moins un coupleur jaune de formule

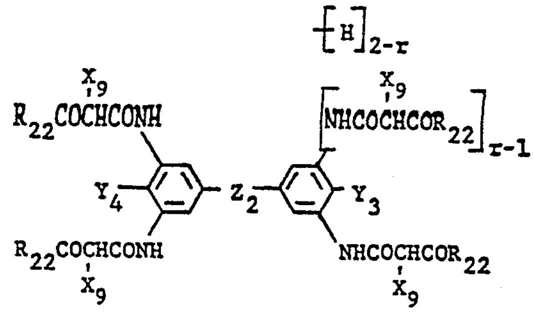

où

$R_{22}$ désigne un alkyle à chaîne droite un ramifiée ayant de 3 à 10 atomes de carbone, cyclopentyle, cyclohexyle, adamantyle, phényle ou phényle substitué par chlore ou brome, alkyle ou alcoxy ayant chacun de 1 à 4 atomes de carbone ou $—O—(CH_2)_rO—$,

$X_9$ désigne hydrogène, chlore ou un reste de formule

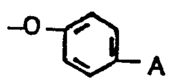 , 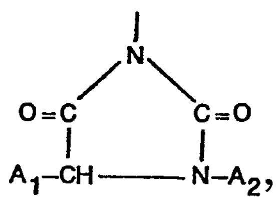 , 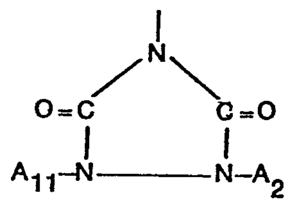 , 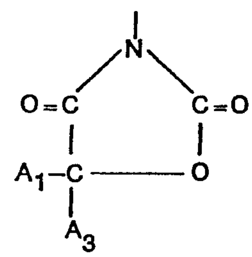 ,

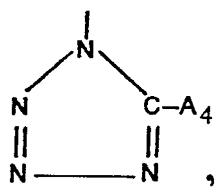 , 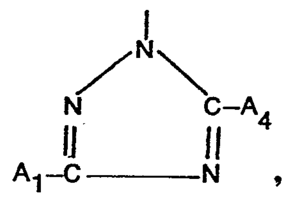 , 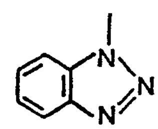 , 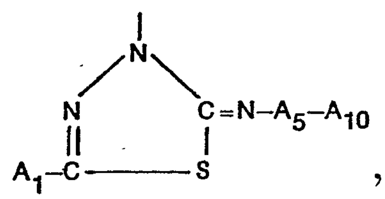 ,

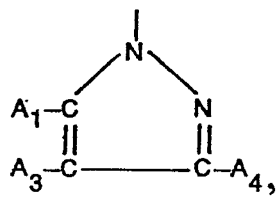 , 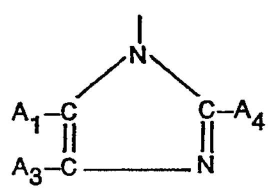 , 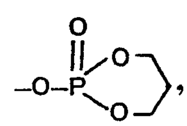 , 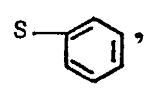 , 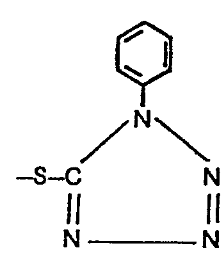 ,

où A désigne COOH, $—NO_2$, $—COOR_{21}$, où $R_{21}$ désigne alkyle ayant de 1 à 4 atomes de carbone, ou le reste de formule

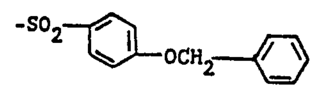

$A_1$ désigne hydrogène, alkyle ayant de 1 à 18 atomes de carbone, aralkyle, aryle, cycloalkyle ayant jusqu'à 4 noyaux cycloalkyle, alcoxy ayant de 1 à 18 atomes de carbone, aryloxy, alkylmercapto ayant de 1 à 18 atomes de carbone, arylmercapto, halogène, trifluorométhyle, cyano, $—NH_2$, mono- ou dialkylamino, les restes alkyle contenant chacun de 1 à 18 atomes de carbone,

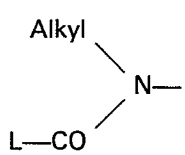

où alkyle renferme de 1 à 5 atomes de carbone,

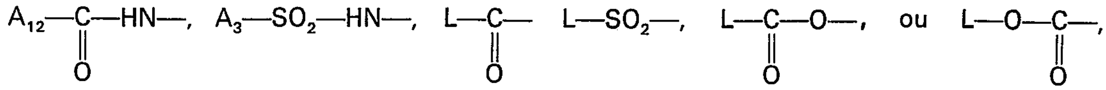

$A_2$ désigne alkyle à chaîne droite ou ramifiée ayant de 1 à 18 atomes de carbone, aralkyle et de préférence benzyle ou bien phényle substitué par alkyle, alcoxy, halogène, —$NH_2$, alkylamino, dialkylamino, acylamino, —COOH, carbalcoxy, carbonamido, sulfo, sulfonamido ou alkylmercapto

$A_3$ désigne alkyle ramifié ou non ramifié ayant de 1 à 18 atomes de carbone, éventuellement substitué par halogène, nitro, cyan, alcoxy ou par des groupes amine primaire, secondaire ou tertiaire, aralkyle, cycloalkyle ayant jusqu'à 4 noyaux cycloalkyle; aryle éventuellement substitué par alkyle, ou alcoxy, chacun ayant de 1 à 4 atomes de carbone, halogène, acylamino, —$SO_3H$, —COOH, sulfonamide ou carboxylamide, sulfonamide ou carboxylamide N— ou N,N-substitués, ester d'acide carboxylique hydroxyle, nitro, amine primaire, secondaire ou tertiaire, mercapto, alkylmercapto, —$SO_2$—L ou —CO—L; pyridyle, furyle, thiényle, perfluoroalkyle, acyle, dialkylamino ayant chacun de 1 à 5 atomes de carbone dans la partie alkyle, alcoxy ayant de 1 à 18 atomes de carbone ou phénoxy;

$A_4$ désigne hydrogène, alkyle ayant de 1 à 18 atomes de carbone, éventuellement substitué, cycloalkyle, cycloalcényle, alcényle, aryle, aralkyle, un reste hétérocyclique, alcoxy, aryloxy, alkylmercapto, amino éventuellement substitué par alkyle, aryle ou acyle, alkylsulfonyle, arylsulfonyle, acyloxy, aminosulfonyle, carboxyamide, sulfonamide, alkylester d'acide carboxylique, nitro, cyan, halogène, uréido éventuellement substitué ou aminosulfonylamino éventuellement substitué; et

$A_5$ désigne —CO— ou —$SO_2^-$;

$A_{10}$ a la signification de $A_3$ sauf —$SO_2$—L ou —CO—L et en outre peut désigner hydrogène à condition que $A_5$ désigne —CO—;

$A_{11}$ alkyle ayant de 1 à 18 atomes de carbone, éventuellement substitué par halogène, amino, cyano ou alcoxy, cycloalkyle, aryle ou aralkyle;

$A_{12}$ désigne hydrogène ou a la signification de $A_3$;

L désigne alkyle ayant de 1 à 18 atomes de carbone, éventuellement substitué par halogène, amino, cyano ou alcoxy; cycloalkyle, aryle, pyridyle, pyrimidyle, furyle ou thiényle et $Y_3$, $Y_4$, $Z_2$ et r ont les significations indiquées dans la revendication 4.

6. Matière d'enregistrement selon la revendication 2, caractérisée par le fait qu'elle contient au moins un coupleur jaune de formule

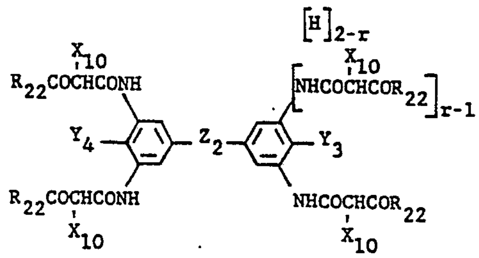

où

$X_{10}$ désigne hydrogène, chlore ou un reste de formule

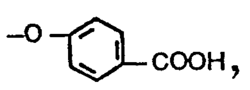

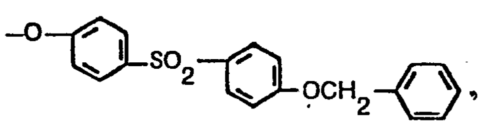

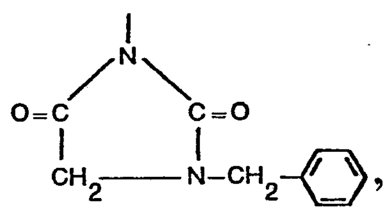

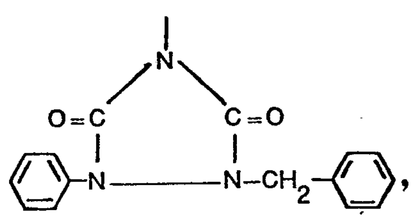

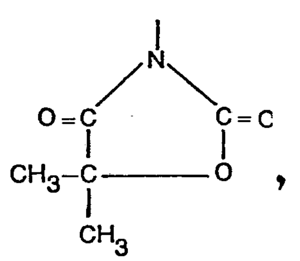

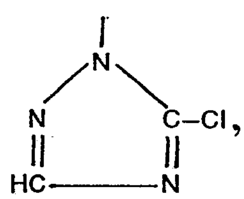

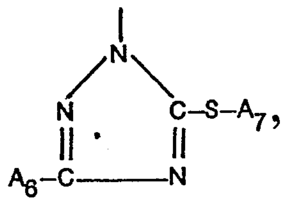, 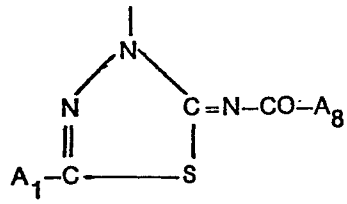 ou 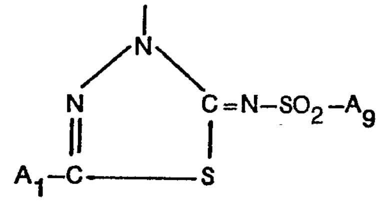

où $A_6$ désigne hydrogène ou alkyle ayant de 1 à 4 atomes de carbone, $A_7$ désigne alkyle ayant de 1 à 12 atomes de carbone, $A_8$ désigne alkyle à chaîne droite ou ramifiée ayant de 1 à 18 atomes de carbone,

$-CH_2Cl$, $-CCl_3$, 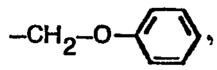, 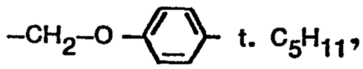,

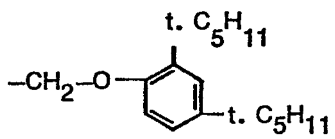, 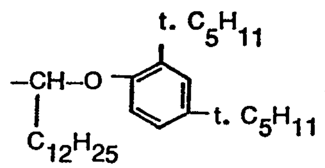, 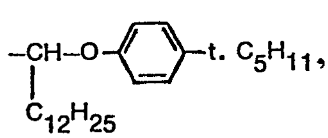,

$-CH_2-O-C_pH_{2p+1}$,

aralkyle, cycloalkyle, phényle ou phényle substitué par

$-C_qH_{2q+1}$, $-OC_qH_{2q+1}$, $-Cl$, $-Br$, $-NHOCC_pH_{2p+1}$,

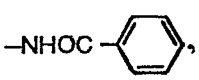, $-SO_2NH_2$, 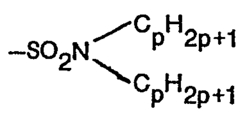, 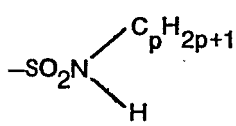, $-CONH_2$,

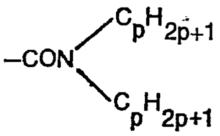, 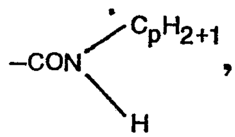, $-SO_2CH_3$, 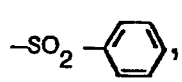, 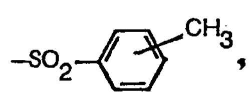,

$-COOH$, $-COOC_pH_{2p+1}$, 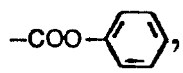, $-COO-C_pH_{2p+1}$, 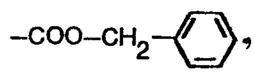, $-OH$,

$-NH_2$, 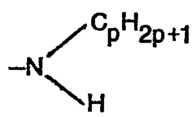, 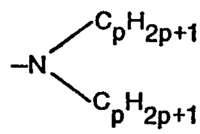, $-SC_pH_{2p+1}$, $-SO_3H$,

$-COC_pH_{2p+1}$ ou 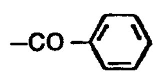 ;

pyridyle, furyle, thiényle, $-C_pF_{2p+1}$, $-COC_pH_{2p+1}$,

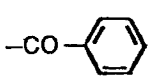, 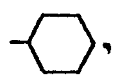, norbornyle, adamantyle,

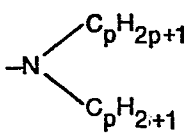, $-O-C_pH_{2p+1}$ ou 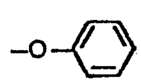

p désigne un nombre de 1 à 5, q désigne un nombre de 1 à 4 et $A_9$ désigne

$-CH_3$, 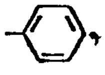, 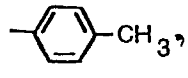, 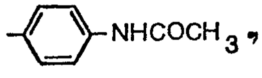,

et

$R_{22}$ désigne un alkyle à chaîne droite ou ramifiée ayant de 3 à 10 atomes de carbone, cyclopentyle, cyclohexyle, adamantyle, phényle ou phényle substitué par chlore ou brome, alkyle ou alcoxy ayant chacun de 1 à 4 atomes de carbone ou $—O(CH_2)_rO—$, et $Y_3$, $Y_4$, $Z_2$ et r ont les significations indiquées dans la revendication 2.

7. Procédé photographique en couleur pour l'obtention d'une image jaune par développement en couleur d'une matière d'enregistrement exposée selon l'image, selon l'une quelconque des revendications 1 à 6.

8. Composés de formule

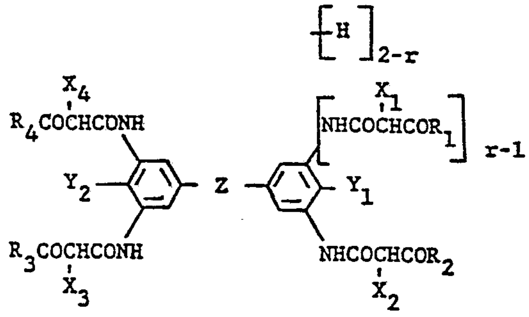

où

$R_1$, $R_2$, $R_3$ et $R_4$ désignent alkyle, cycloalkyle ou aryle,

$X_1$, $X_2$, $X_3$ et $X_4$ sont des restes éliminables au cours d'une réaction de copulation, et

$Y_1$ et $Y_2$ désignent halogène, alkyle, alcoxy, alkylmercapto, —CN, —COOH, carbaloxy, $—NH_2$, $—NHR_5$, $—NR_5R_6$ ou $NHCOR_5$, où $R_5$ et $R_6$ désignent alkyle ou phényle,

Z désigne alkylène à chaîne droite ayant de 1 à 20 atomes de carbone, éventuellement substitué par alkyle, hydroxyalkyle, alcoxyalkyle, aminoalkyle, acylaminoacyle ou halogénoalkyle ayant chacun de 1 à 10 atomes de carbone dans la partie alkyle et 1 à 5 atomes de carbone dans la partie alcoxy et acyle, hydroxyle, halogène, alcoxy ayant de 1 à 5 atomes de carbone, amino ($—NH_2$), N-alkylamino et N,N-dialkylamino, chacun ayant de 1 à 5 atomes de carbone dans la partie alkyle, mercapto (—SH) et/ou alkylmercapto ayant de 1 à 5 atomes de carbone, Z peut en outre désigner cycloalkylène ayant de 3 à 12 atomes de carbone,

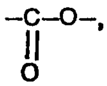, 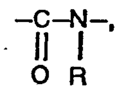, 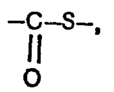, $-SO_2-$, $-SO_2-O-$, 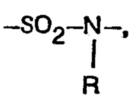, 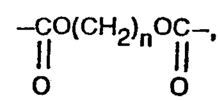,

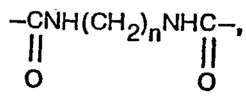, $-SO_2O(CH_2)_nOSO_2-$, $-SO_2NH(CH_2)_nNHSO_2-$, 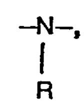,

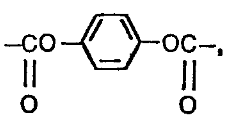, 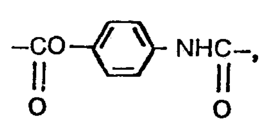, 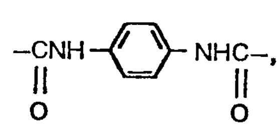, 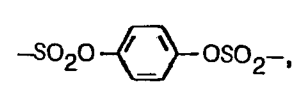,

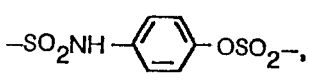, 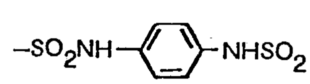, 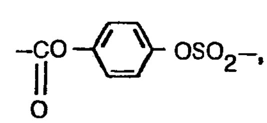,

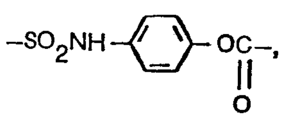, 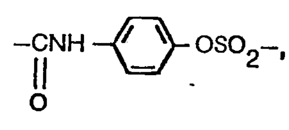,

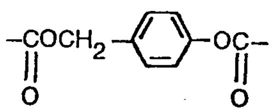 ou 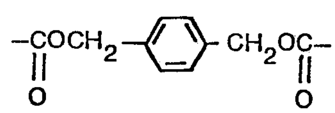

où R désigne hydrogène, alkyle ayant de 1 à 10 atomes de carbone, éventuellement substitué par halogène, hydroxyle ou acyle ayant de 2 à 5 atomes de carbone et n désigne un nombre entier de 2 à 20 et r désigne 1 ou 2.

9. Procédé de préparation de composés selon la revendication 8, caractérisé par le fait qu'on réduit des composés trinitro ou tétranitro de formule

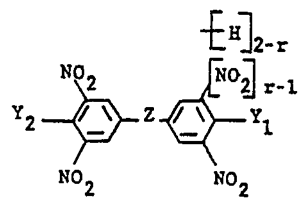

où $Y_1$, $Y_2$, Z et r ont les significations indiquées dans la revendication 8, en composés amino correspondants, qu'on fait réagir avec 3 ou 4 moles d'esters de formules $RCOCH_2COOAlk$, où R a la signification de $R_1$ à $R_4$ dans la revendication 8, éventuellement on remplace un atome d'hydrogène dans les groupes $—CH_2—$ par un atome d'halogène et qu'on fait réagir les composés ainsi obtenus avec des sels de formule $M^{\oplus}A^{\ominus}$ où $M^{\oplus}$ est un cation et X a la signification de $X_1$ à $X_4$ dans la revendication 8.

10. Utilisation de composés selon la revendication 8 comme coupleurs jaunes dans des matières d'enregistrement photographiques en couleur.